# EUROPÄISCHE PATENTSCHRIFT

(11) **EP 1 275 219 B1**
(45) Veröffentlichungstag und Bekanntmachung des Hinweises auf die Patenterteilung: **04.06.2003**
(21) Anmeldenummer: 00991057.1
(22) Anmeldetag: 11.12.2000
(51) Int. Cl.: H04L 12/00, H04L 1/00, G08C 17/02

(54) **Funkstrecke mit Sender und Empfänger sowie Verfahren zu deren Betrieb**
Radio Path having Transmitter and Receiver, and Method for Operating Same
Ligne hertzienne avec émetteur et récepteur, et procédé d'utilisation

(30) Priorität: 09.12.1999 DE 19959545
(43) Veröffentlichungstag der Anmeldung: 15.01.2003
(73) Patentinhaber: Iar Systems AG, 85599 Parsdorf (DE)
(72) Erfinder: DE MIER, Eduardo, 85599 Parsdorf (DE)
(74) Vertreter: Schweiger, Martin, Dipl.-Ing.
(86) Internationale Anmeldenummer: DE0004411
(87) Internationale Veröffentlichungsnummer: WO01043350

(56) Entgegenhaltungen:
- EP-A- 0 698 974
- EP-A- 0 962 904
- WO-A-97/25699
- BLACKMORE P A ET AL: "CODING TECHNIQUES TO IMPROVE THE RELIABILITY OF EMCON TRANSMISSIONS" ANNUAL MILITARY COMMUNICATIONS CONFERENCE,US,NEW YORK, IEEE, Bd. 15TH, 22. Oktober 1996 (1996-10-22), Seiten 447-452, XP000697320 ISBN: 0-7803-3683-6

## Beschreibung

Die Erfindung betrifft eine Funkstrecke insbesondere mit mehreren Sendern und mit einem Empfänger, bei der ein aus Datenpaketen bestehender Datenstrom von den Sendern zu dem Empfänger übertragbar ist.

Bei solchen Funkstrecken ist problematisch, daß häufig Übertragungsfehler auftreten, beispielsweise wenn alle Sender gleichzeitig senden. Dann werden jeweils einzelne Datenpakete gestört, so daß keine ordnungsgemäße Übertragung des Datenstroms zwischen den Sendern und dem einzigen Empfänger zustande kommt.

Zur Lösung dieses Problems könnte eine bidirektionale Datenübertragung auf der Funkstrecke vorgesehen werden, bei der gewährleistet wird, daß zu einem bestimmten Zeitpunkt immer nur je ein Sender mit je einem Empfänger kommuniziert. Dieses Verfahren ist jedoch energieaufwendig, so daß es für batteriebetriebene Sender und Empfänger nicht anwendbar ist.

Die EP 0 698 974 A1 zeigen ein Übertragungssystem mit einem Sender und einem Empfänger, bei dem Datenpakete in einem Zeitmultiplexverfahren übertragen werden. Dabei werden fehlerfreie Datenpakete aus einer Vielzahl von fehlerbehaftet übertragenen Datenpaketen rekonstruiert.

Der Aufsatz "Coding techniques to improve the reliability of emcon transmissions" von Perry A. Blackmore und Stephen C. Cook in annual military communications conference, US, New York, IEEE, Band 15 vom 22. Oktober 1996, Seite 447-452, ISBN 0-7803-3683-6 veranschaulicht eine bessere Datenübertragung, bei dem ein fehlerfreies Datenpaket aus bis zu vier fehlerbehafteten Datenpaketen rekonstruiert wird. Hierzu werden verschiedene Kombinationen von bekannten Rekonstruktionsverfahren veranschaulicht und miteinander verglichen.

Es ist Aufgabe der Erfindung, einen Sender und einen Empfänger für eine Funkstrecke sowie ein Verfahren zu deren Betrieb bereitzustellen, die eine sichere und energiesparende Datenübertragung ermöglichen.

Diese Aufgabe wird durch den Gegenstand der unabhängigen Ansprüche gelöst. Vorteilhafte Weiterbildungen ergeben sich aus den jeweiligen Unteransprüchen.

Gemäß der Erfindung weist der Sender insbesondere eine Eingangseinheit zur Eingabe bzw. Generierung je eines Datenpakets bzw. Datensatzes oder Datums sowie eine Sendersteuereinheit zur Verarbeitung des Datenpakets und zum einer sich aus dem Inhalt des Datenpakets ergebenden vorbestimmten Prüfcodierung in das Datenpaket auf. Weiterhin ist eine von der Sendersteuereinheit betätigbare Sendeeinheit zur Aussendung des Datenpakets vorgesehen, wobei der Sender so ausgebildet ist, daß die Datenpakete regelmäßig hintereinander aussendbar sind.

Die Signale eines solchen Senders lassen sich besonders einfach von einem Empfänger abtasten. Dabei braucht im einfachsten Fall mit nur einem Sender nur eine einmalige Aktivität des Senders festgestellt werden, um die weiteren Aktivitäten des Senders vorherzusagen. In Systemen mit mehreren Sendern lassen sich die Sendezeitpunkte der Sender ebenso einfach vorhersagen, wenn alle Sendewiederholzeiten der in der Funkstrekke vorhandenen Sender vorher dem Empfänger mitgeteilt wurden. Der Empfänger braucht dann nur noch die Aktivitäten auf der Funkstrecke feststellen und versuchen, zu den möglichen nachfolgenden Zeitpunkten und bei Vielfachen der Wiederholungszeiten Datenpakete zu empfangen. Ein derart getaktetes Übertragungsverfahren läßt sich vorteilhaft bei solchen Funkstrecken einsetzen, bei denen es nicht von besonderer Bedeutung ist, ob jedes Datenpaket des Datenstroms übertragen wird oder nicht. Somit läßt sich die Erfindung besonders gut bei Funkstrecken zur Übertragung von Meßwerten einsetzen, bei denen sich der übertragene Meßwert im Vergleich mit der Ubertragungshäufigkeit der Meßwerte nur sehr langsam ändert.

Durch das Vorsehen einer sich insbesondere aus dem Inhalt des jeweils übertragenen Datenpakets ergebenden vorbestimmten Prüfcodierung im Datenpaket lassen sich dabei auf einfache Weise ordnungsgemäß übertragene Datenpakete von defekten Datenpaketen unterscheiden, so daß auf der Seite des Empfängers nur fehlerfreie Datenpakete weiter verarbeitet werden brauchen.

Entsprechend zum erfindungsgemäßen Sender hat der erfindungsgemäße Empfänger der Funkstrecke eine Empfangseinheit zum Empfang von Datenpaketen sowie eine mit der Empfangseinheit verbundene Empfängersteuereinheit, mit der die empfangenen Datenpakete verarbeitbar und ausgebbar sind. Die Empfängersteuereinheit kann dabei je eine Prüfcodierung eines Datenpakets ermitteln, wobei aus einem Vergleich der Prüfcodierung mit dem Inhalt des Datenpakets bestimmbar ist, ob ein fehlerfreies oder ein fehlerbehaftetes Empfangen des Datenpakets vorliegt. Bei einem Empfang eines fehlerbehafteten Datenpakets wird das betreffende Datenpaket verworfen.

Der erfindungsgemäße Empfänger ist so ausgebildet, daß Zeitpunkte für einen zu erwartenden Eingang je eines Datenpakets bestimmbar sind. Dies kann beispielsweise durch zeitweises Abtasten aller Aktivitäten auf der Funkstrecke erfolgen, wobei ein besonders energiesparender Einsatz des Empfängers dann ermöglicht wird, wenn lediglich abgetastet wird, ob überhaupt eine Aktivität vorliegt. In vorgegebenen zeitlichen Abständen wird dann vom Zeitpunkt des Ertastens einer Aktivität aus versucht, Datenpakete zu empfangen. Eine solche einfache Lösung bietet sich insbesondere dann an, wenn die zeitlichen Abstände zwischen den von den jeweiligen Sendern ausgesendeten Datenpaketen dem Empfänger bekannt sind.

In einer Weiterbildung der Erfindung ist die Sendersteuereinheit so ausgebildet, daß wenigstens ein Duplikat des Datenpakets erstellbar ist, wobei das Datenpaket und/oder das Duplikat jeweils eine Typinformation aufweisen. Mit einer solchen Typinformation kann von dem Empfänger erfaßt werden, ob es sich bei dem empfangen Datenpaket um ein Datenpaket selbst oder um ein Duplikat eines Datenpakets handelt.

Der Sender ist so ausgebildet, daß je ein Datenpaket sowie ein Duplikat regelmäßig hintereinander aussendbar sind, wobei ferner die zeitliche Versetzung zwischen je einem Datenpaket und dessen Duplikat gemäß einer vorbestimmten Versetzungsregel und einer Versetzungsinformation variierbar ist. Die Versetzungsinformation kann man dabei in einem Datenpaket oder in einem Duplikat vorgesehen sein. Durch das Aussenden eines oder mehrerer Duplikate des ausgesendeten Datenpakets kann sich der Empfänger beim Empfangen eines defekten Datenpakets die in dem Datenpaket enthaltenen Daten auch aus dem nachfolgend ausgesendeten Duplikat beschaffen. Beim Aussenden mehrerer Duplikate stehen dem Empfänger dann sogar mehrere Möglichkeiten zur Rekonstruktion der defekten Daten des Datenpakets zur Verfügung. Die zeitliche Versetzung zwischen je einem Datenpaket und dessen Duplikaten ist gemäß der Versetzungsregel sowohl dem Sender als auch dem Empfänger bekannt. In einer besonders einfachen Ausgestaltung der Erfindung kann der Empfänger schon aufgrund einer einzigen Versetzungsinformation in einem einzigen Datenpaket den zu erwartenden Eingangszeitpunkt aller folgenden Duplikate vorhersagen. Dadurch ergibt sich ein besonders sicherer Betrieb, weil nur wenige Informationen über die zeitliche Abfolge des Sendens von Datenpaketen und Duplikaten übertragen werden müssen.

Die Versetzungsinformation kann aus einer vorgegebenen Zählfolge generiert werden, und zwar insbesondere unter Verwendung eines Zählerstands, mit dem die vom Sender gesendeten Datenpakete gezählt werden. Dabei ist es empfängerseitig nicht notwendig, wirklich jedes vom Sender gesendete Datenpaket zum empfangen, weil diesem die Zeitpunkte, zu denen Datenpakete ausgesendet werden sollen, bekannt sind. Somit kann der Empfänger den Zähler auch betreiben, ohne wirklich alle gesendeten Datenpakete empfangen zu müssen. Trotzdem bleibt für ihn die relative Lage zwischen Duplikaten und Datenpaketen rekonstruierbar, weil der von ihm geführte Zählerstand stets aktuell ist.

Durch das Vorsehen einer Typinformation in dem Datenpaket bzw. in dem Duplikat ist es dem Empfänger dabei auf einfache Weise möglich, zu jedem Zeitpunkt eine Unterscheidung zwischen Datenpaketen und Duplikaten vorzunehmen. Dies bewährt sich besonders beim Einrasten eines erfindungsgemäßen Senders und eines erfindungsgemäßen Empfängers zu Beginn des Betriebs der Funkstrecke.

Weiterhin kann je ein Datenpaket bzw. dessen Duplikat mit einer dem zugehörigen Sender zugeordneten Identitätsinformation versehbar sein, die eine Information über die Art bzw. Bedeutung der Datenpakete enthalten kann. So kann innerhalb der Funkstrecke eine bestimmte Sub-ID des jeweiligen Senders einer Übertragungsrate oder einem Übertragungsraster der Datenpakete zugeordnet sein. Außerdem kann einer bestimmten Sub-ID eine Bedeutung der mit den Datenpaketen übermittelten Daten zugeordnet sein. So lassen sich beispielsweise übertragene Geschwindigkeitsdaten auf einfache Weise von übertragenen Strekkendaten unterscheiden. Die Identitätsinformation kann auch einen Teil einer eindeutigen Information über den jeweiligen Sender beinhalten. Hierdurch läßt sich innerhalb der Funkstrecke auf besonders einfache Weise feststellen, ob die in der Funkstrecke eingeschalteten Sender auch wirklich zu dem vorgesehenen System gehören. Zu einem anderen System mit einer anderen Funkstrecke gehörende Sender lassen sich dadurch auf besonders einfache Weise erkennen, wobei nachfolgend der Empfang der unerwünschten Daten von diesen Systemen unterdrückbar ist.

Gemäß dem erfindungsgemäßen Verfahren sind zum Empfangen eines Datenpakete aufweisenden Datenstroms ein Such-Modus sowie ein Ubertragungsmodus vorgesehen. Im Such-Modus werden dabei die folgenden Schritte ausgeführt:
- Abtasten des Datenstroms auf das Verhandensein von Datenpaketen,
- Vorbestimmen von Zeitpunkten für einen zu erwartenden Eingang je eines weiteren Datenpakets.

In einem solchen Such-Modus reicht es im einfachsten Fall aus, durch Datenpakete verursachte Aktivitäten auf der Funkstrecke abzutasten. Zeitpunkte für den Eingang weiterer Datenpakete nach einem zuerst abgetasteten Datenpaket ergeben sich dann aus Vielfachen einer vorbekannten Abstandszeit zwischen den jeweiligen Datenpaketen, die zu dem Zeitpunkt des Eintreffens des ersten Datenpakets hinzu addiert werden. Im Übertragungsmodus wird dann jeweils ein Datenpaket selektiv ausgewertet, wobei aus einem Vergleich der Prüfcodierung mit dem Inhalt des Datenpakets bestimmt wird, ob ein fehlerfreies oder ein fehlerbehaftetes Empfangen des Datenpakets vorliegt. Bei dem erfindungsgemäßen selektiven Auswerten kann ein Empfänger, der das erfindungsgemäße Verfahren ausführt, selektiv aus- oder eingeschaltet werden.

Das erfindungsgemäße Verfahren läßt sich vorteilhaft dadurch verbessern, daß der Datenstrom auf das Vorhandensein von zu den Datenpaketen gehörenden Duplikaten abgetastet wird. Wenn anschließend eine Versetzungsregel für eine zeitliche Versetzung zwischen Datenpaketen und Duplikaten aus wenigstens einer den Datenpaketen und/oder deren Duplikaten entnommenen Versetzungsinformation bestimmt wird, ergibt sich eine vergrößerte Ubertragungssicherheit. Dann kann bei einem Empfangen eines defekten Datenpakets die übertragene Information immer noch dem Duplikat entnommen werden.

Eine besonders energiesparende Betriebsweise der erfindungsgemäßen Vorrichtung ergibt sich dann, wenn die Sendeeinheit zwischen einem eingeschalteten Zustand und einem Zustand mit verringerten Energieverbrauch umschaltbar ist. Vorzugsweise wird die Sendeeinheit selektiv in eingeschaltetem Zustand gehalten, wenn Datenpakete bzw. Duplikate gesendet werden. Zu Zeitpunkten, an denen keine Datenpakete bzw. Duplikate gesendet werden, wird die Sendeeinheit in dem Zustand mit verringerten Energieverbrauch oder sogar ganz ausgeschaltet gehalten. Dadurch läßt sich in großem Maße Sendeenergie einsparen.

Genauso kann auch die Empfangseinheit des Empfängers zwischen einem eingeschalteten Zustand und einem Zustand mit verringerten Energieverbrauch umgeschaltet werden, wobei die Empfangseinheit zu Zeitpunkten, an denen ein Empfang eines Datenpakets bzw. eines Duplikats zu erwarten ist, in eingeschalteten Zustand haltbar ist. Dagegen wird die Empfangseinheit zu Zeitpunkten, an denen kein Empfang zu erwarten ist, in dem Zustand mit verringerten Energieverbrauch gehalten. Gemäß der Erfindung kann die Empfangseinheit selektiv für eine vorgegebene Zeit ausschaltbar sein, beispielsweise während eines Hold-Modus, in dem die Funkstrecke nicht betrieben werden muß. Unabhängig davon ist es denkbar, daß selbst bei ausgeschalteter Empfangseinheit die empfängerseitig durchgeführte Berechnung der zu erwartenden Empfangszeiten für Datenpakete bzw. deren Duplikate weiterhin ausgeführt wird, so daß schon durch einfaches Ingangsetzen der Empfangseinheit die Funkstrecke wieder in synchronisiertem Betrieb betreibbar ist.

In einer besonderen Ausbildung ist der Empfänger so ausgebildet, daß aus je einem Datenpaket bzw. aus je einem Duplikat eine dem Sender des Datenpakets bzw. des Duplikats zugeordnete Identitätsinformation decodierbar ist. Dies wird insbesondere zur Bestimmung des zeitlichen Abstands zwischen zwei Datenpaketen verwendet, wobei dieser von Datenpaketen mit übereinstimmenden Identitätsinformationen bestimmt wird. Dadurch läßt sich eine besonders schnelle und genaue Synchronisation der Funkstrecke erreichen.

Zum Bestimmen der Zeitpunkte für einen zu erwartenden Eingang eines Datenpakets kann ein von der Empfangseinheit an die Empfängersteuereinheit geliefertes Signal in einem Such-Modus des Empfängers auch wiederholt selektiv ausgewertet werden. Eine selektive Auswertung kann beispielsweise darin bestehen, daß das von der Empfangseinheit abgegebene Signal in regelmäßigen Abständen auf Aktivitäten hin überprüft wird. Mit dem erfindungsgemäßen Empfänger werden Zeitpunkte für einen zu erwartenden Eingang je eines Datenpaketes aus einem Anfangszeitpunkt beim Eingang eines ersten Datenpakets sowie aus einer vorbestimmten, insbesondere aus dem Inhalt oder aus der zeitlichen Lage des ersten Datenpakets rekonstruierbaren Abstandszeit errechnet. Dabei ist auch möglich, aus dem empfangenen Datenpaket direkt die Abstandszeit zwischen zwei aufeinanderfolgenden Datenpakten zu rekonstruieren, beispielsweise indem eine bestimmte Kennung wie die Sub-ID ausgewertet wird. Dadurch ergibt sich eine schnelle und ökonomische Berechnung der regelmäßigen Abstandszeit zwischen zwei aufeinanderfolgenden Datenpaketen.

Abweichend von der vorstehend erläuterten Methode zum Auffinden der Abstandszeit zwischen zwei aufeinanderfolgenden Datenpaketen können Zeitpunkte für einen zu erwartenden Eingang je eines Datenpakets auch aus einer Abstandszeit zwischen zwei Datenpaketen errechnet werden, die aus einem Anfangszeitpunkt beim Eingang eines ersten Datenpakets sowie aus einem Wiederholungszeitpunkt beim Eingang eines weiteren Datenpakets ermittelt wird.

Die Erfindung ist auch in einem kombinierten Sende/Empfangsmodul für eine Funkstrecke verwirklicht, das einen erfindungsgemäßen Sender und/oder einen Empfänger aufweist. Der Empfänger und der Sender können zur Vermeidung von Kollisionen von Datenpaketen oder deren Duplikaten auch betätigbar miteinander verbunden sein. Dabei kann der Empfänger beim Abtasten eines von einem weiteren Sender gesendeten Datenpakets den mit ihm verbundenen Sender zeitweilig unterdrücken oder zum Aussenden von Datenpaketen mit verändertem zeitlichen Abstand veranlassen.

Die Erfindung umfaßt auch Verfahren zum Betreiben einer Funkstrecke gemäß der Erfindung, die die erfindungsgemäßen Verfahren zum Aussenden eines Datenstroms sowie zum Empfang eines Datenstroms beinhalten.

Mit der erfindungsgemäßen Funkstrecke ist ein Langzeitbetrieb von Sender und Empfänger möglich, wenn diese aus Batterien gespeist werden. Dadurch ergeben sich Einsatzmöglichkeiten insbesondere im Zusammenhang mit Sportuhren, die Geschwindigkeits- und Herzschlagssensoren auswerten. Das erfindungsgemäße System ist vorteilhaft in unidirektionalen als auch bidirektionalen Systemen einsetzbar, die jeweils aus einem Empfänger und mehreren Sendern bestehen. Das erfindungsgemäße System weist eine hohe Immunität gegen Sender gleichartiger benachbarter Systeme und gegen Kollisionen der Sender eines Systems untereinander auf. Es ergibt sich weiterhin eine hohe Störfestigkeit gegenüber Fremdeinstrahlung. Schließlich ist aus der Sicht eines Endanwenders eine einfache Inbetriebnahme bzw. ein unkomplizierter Betrieb gewährleistet.

Bei dem erfindungsgemäßen Protokoll wird die Information in der Form von wiederkehrenden Sendepakten mit typischerweise 8 bis 16 Datenbits sowie diversen Steuer- und Prüfbits übertragen. Zusätzlich können durch einen Zahlensequenzgenerator über den Zeitbereich hinweg verstreute Sendepakete redundant zu den regelmäßigen, im fixen Abstand aufeinanderfolgenden Sendepaketen bereitgestellt werden. Nach einer Synchronisationsphase ist die Lage aller ankommenden Pakete für den Empfänger berechenbar. Durch gezieltes An- und Ausschalten des Empfängers und des Senders wird ein niedriger Stromverbrauch erreicht.

Die Erfindung läßt sich besonders vorteilhaft bei Anwendungen einsetzen, die eine unidirektionale Übertragung von Daten bei einer niedrigen Datenrate erfordern. Dabei ergeben sich Vorteile im Langzeitbetrieb wie beispielsweise bei der Überwachung von Meßdaten, die sich mit einer Rate im Bereich von wenigen Hertz ändern. Nach einer kurzen vorhergehenden Synchronisationszeit ist bei der Erfindung eine Übertragung von Daten möglich. Ein Kurzzeitbetrieb wie beispielsweise die Fernsteuerung von Geräten kann mit zusätzlichem Verfahrensaufwand erreicht werden.

Systeme mit mehreren Sendern, die trotz Auftreten der Kollisionen zwischen Datenpaketen und anderer Störungen mit möglichst gleichmäßiger Rate von einem Empfänger empfangen werden sollen, lassen sich durch die erfindungsgemäßen Redundanzfunktionen bei einem gleichzeitig extrem sparsamen Langzeitbatteriebetrieb erreichen. Hierzu gehören beispielsweise Sportcomputer, medizinische Geräte zur Patientenüberwachung, Alarmanlagen, Überwachungssysteme im Industrie- und Heimanwenderbereich sowie Meßdatenübertragungen.

Mit der Erfindung ergibt sich gerade bei Sportcomputern ein verbessertes Ubertragungsprotokoll, mit dem sich ein einziger Empfänger für alle Sender eines Systems verwenden läßt. Alle Sender senden dann auf einer gemeinsamen Frequenz, wobei sich eine maximale Zahl von 4 bzw. 6 oder 8 Sendern bewährt hat. Mit der Erfindung läßt sich eine hohe Übertragungsrate in einem vorgegebenen Zeitrahmen erreichen, wobei Kollisionen zwischen den verschiedenen Sendern bzw. Sensoren eines Systems vermieden werden. Die erfindungsgemäße Funkstrecke hat dabei eine hohe Störfestigkeit gegenüber atmosphärischen Störungen und Sender anderer Systeme. Aus der Erfindung resultiert ferner eine sehr niedrige mittlere Aktivität der Empfänger- und Senderbausteine, so daß sich ein niedriger Stromverbrauch der Funkstrecke erreichen läßt.

Die Erfindung umfaßt auch ein Protokoll für eine Datenübertragung, bei dem Datenwörter vor dem Senden in ein oder mehrere Datenpakete umgewandelt werden. Jeder Sender der Funkstrecke hat eine jeweils unterschiedliche Sub-ID, die den jeweiligen Sensortyp innerhalb eines Systems angibt. So lassen sich Sensoren für einen Herzschlag, für eine Radumdrehung oder für eine Pedalfrequenz anhand der Sub-ID unterscheiden. Außerdem hat jedes Sendermodul unabhängig von der Sub-ID eine einmalig vorkommende ID oder auch Seriennummer. Diese wird beispielsweise bei einem Fertigungstest in einem nichtflüchtigen Speicherbereich des Senders geschrieben oder beim Einlegen einer neuen Batterie nach einem Zufallsprinzip ermittelt. Jeder Sub-ID ist eine bestimmte, fixe Paketwiederholzeit bzw. Paketfrequenz zugewiesen. Die Paketfrequenz ist von der jeweiligen Frequenz, mit der die Meßwerte eintreffen oder sich ändern, unabhängig. Parallel zum Begriff Paketfrequenz kann auch eine Bezeichnung "Time-Slot" benutzt werden. Es handelt sich dabei um ein Raster aus Zeitabständen, die der Paketlänge entsprechen. Der Abstand zwischen zwei Paketen eines Senders beträgt immer eine ganzzahlige Anzahl an Timeslots. Der Paketabstand der einzelnen Sub-IDs differiert immer um eine gerade Zahl.

Zur Kollisionsvermeidung innerhalb eines Systems einer Funkstrecke soll erreicht werden, daß für jeden Sender die Kollision seiner Pakete mit Paketen eines anderen Senders des gleichen Systems nicht länger als ein Paket in Folge unterbrochen ist. Die erfindungsgemäßen Sender haben abhängig von der Sub-ID eine unterschiedliche fixe Paketfrequenz, die sie durch eine Kodierung innerhalb des Systems erhalten. Diese Kodierung variiert in Stufen, und zwar so, daß sich die Periodenlänge der Paketfrequenz von Sender zu Sender jeweils um die zweifache Paketlänge erhöht. Es läßt sich leicht nachvollziehen, daß dann bei zwei Sendern, die zwei unterschiedliche Paketfrequenzen haben, tatsächlich keine zwei Pakete in Folge kollidieren können.

Grundsätzlich gilt jedoch, daß bei einer Anzahl N Sendern im schlechtesten Fall N-1 Pakete eines der Sender in Folge gestört werden können. Dieser Fall ist jedoch unwahrscheinlich und in der Realität von geringerer Bedeutung. Um eine vorgeschriebene maximale Übertragungszeit nicht zu überschreiten, könnte man nun die Häufigkeit der gesendeten Pakete mit N multiplizieren. Bei vier Sendern müßten in diesem Fall viermal mehr Pakete pro Sekunde übertragen werden als bei einer "normalen" Datenübertragung. Dies bedingt jedoch einen erhöhten Stromverbrauch bei Sender und Empfänger. Gemäß der Erfindung wird ein ähnlicher Effekt mit einem verringerten Aufwand erzielt.

Aus der Summe aller Paketfrequenzen und der jeweiligen Paketdauer errechnet sich der Durchsatz der gesamten Zeitscheibe mit Sendepaketen. Dieser Parameter, den man auch "Belegung" nennen kann, gibt ein Maß über die Festigkeit des Systems gegenüber systemfremden Störungen an. Je mehr Pakete auf der Funkstrecke unterwegs sind und je länger diese jeweils sind, desto höher ist auch die Wahrscheinlichkeit, daß ein kurzer Störimpuls mit einem dieser Pakete kollidiert.

Zur Kollisionsvermeidung zwischen zwei Sendern benachbarter, jeweils überstrahlender Funkstrecken kann zusätzlich zu den Fixpaketen, die mit jeweils konstanter Frequenz ausgesendet und empfangen werden, bei jedem Sender jeweils zwischen zwei Fixpaketen noch ein Paket mit variabler Lage bzw. ein Redundanzpaket ausgesendet werden. Der Time-Slot, in den das Redundanzpaket gelegt wird, wird durch einen Zahlensequenzgenerator bestimmt, dessen Sequenz abhängig von der ID des jeweiligen Senders sein kann. Der Algorithmus für die Zahlensequenz ist sowohl dem Sender als auch dem Empfänger bekannt. Nach einer Synchronisationszeit sind auch die jeweiligen IDs der einzelnen Sender im Empfänger abgelegt, so daß auch diese als Parameter in der Zahlensequenz verwendet werden können. Somit kann der Sender im voraus berechnen, in welchem Time-Slot das nächste Redundanzpaket zu erwarten ist und dementsprechend die Empfangseinrichtung zeitgenau einschalten. Eine jeweils in einem Datenpaket übertragene Startinformation für den Zahlensequenzgenerator kennzeichnet einen Nulldurchgang der Zahlensequenz, so daß Sender und Empfänger anhand dieses Datenpakets synchronisierbar sind.

Die ID des Senders wird periodisch innerhalb der Datenpakete übertragen, und zwar jeweils Stück für Stück. Es brauchen dabei insgesamt nur so viele Bestandteile der Seriennummer übertragen werden, wie zur Individualisierung der vom Zahlensequenzgenerator durchlaufenden Zahlensequenz benutzt werden. Dies beschleunigt die Übertragung der ID bzw. Seriennummer des Senders.

Beim Einrasten des Empfängers mit einem Sender und beim Abscannen aller vorhandenen Sub-IDs des Systems wird es angestrebt, den Empfängerbaustein möglichst selten einzuschalten, um zur Schonung einer Batterie den Stromverbrauch unterhalb eines vorgegebenen Werts zu halten. Wenn alle Sender aktiv sind, befindet sich der Empfänger im Grundzustand. Sobald der Empfänger durch den Anwender aktiviert wird, beginnt er über die Länge einer Periode der Paketfrequenz in kurzen Abständen nach Sendeaktivität zu suchen. Der "Kamm", der dabei gebildet wird, muß so schmal sein, daß keine unbemerkten Pakete zwischen den einzelnen "Zinken" liegen können. Ein einzelner Peak zum Suchen von Sendeaktivität ist dabei kürzer als die Länge eines Datenpakets. Durch die grobe Lage der festgestellten Sendeaktivitäten kann ermittelt werden, wo nachfolgend nach Sendepaketen zu suchen ist.

Im nächsten Durchgang wird versucht, an den vorausberechneten Zeitpunkten vollständige Sendepakete zu empfangen. Sobald ein Fixpaket passend zur jeweils gesuchten Sub-ID des Empfängers korrekt empfangen werden konnte, ist der Empfänger in der Lage, sich auf die fixen Pakete dieses Senders zu synchronisieren. Anschließend wird die Seriennummer aus den einzelnen, seriell übertragenen Bits der Seriennummer in verschiedenen Datenpaketen rekonstruiert. Damit hat der Empfänger schließlich alle Informationen zu Verfügung, um auch die Redundanzpakete nötigenfalls zielsicher aufzufinden. Falls beim Durchkämmen der Zeitscheibe keine Aktivität in der Funkstrecke festgestellt wurde, wird der Vorgang noch einmal oder zweimal wiederholt und dann abgebrochen.

Bevor ein Empfänger einer Funkstrecke weiß, welche Sender der Funkstrecke aufgrund ihrer ID zu seiner Funkstrecke zu rechnen sind, muß er die IDs der jeweiligen Sender wenigstens einmal korrekt empfangen und dauerhaft ablegen. Dazu wird ein entsprechender Modus im Empfänger angewählt und gleichzeitig gewährleistet, daß alle Sender des eigenen Systems aktiv sind. Außerdem muß sichergestellt sein, daß keine Sender eines zweiten, gleichartigen Systems übersprechen. In einem solchen Zustand können die IDs zuverlässig angelernt werden.

Im Normalbetrieb schaltet sich der erfindungsgemäße Empfänger zum jeweiligen Eingangszeitpunkt der Fixpakete ein und beachtet die Redundanzpakete nicht. Wenn aufgrund von Fremdstörungen oder Kollisionen in der Funkstrecke ein Fixpaket fehlerhaft oder nicht empfangen werden kann, versucht der Empfänger, das fehlende Fixpaket durch das nächstfolgende Redundanzpaket oder weitere Redundanzpakete zu ersetzen. Dies wird unter Zuhilfenahme der beidseitig bekannten Zahlensequenz für die Berechnung der zeitlichen Lage der Redundanzpakete erreicht. Wenn die Störungen der Funkstrecke wieder aufhören, beschränkt sich der Empfänger wieder auf den Empfang von Fixpaketen. Dies hält den Stromverbrauch niedrig.

Beim Einrasten zwischen Sender einer Funkstrecke und dem Empfänger oder beim Aufwachen aus einem Modus mit verringertem Energieverbrauch kann der Empfänger auch auf die Sender eines Nachbarsystems reagieren. Über die in einem besonderen Modus eingelernten IDs der Sender des Systems kann jedoch festgestellt werden, ob die beim Einrasten aufgefundenen Sender zum eigenen System gehören oder nicht.

Der mittlere Stromverbrauch im Empfänger errechnet sich aus der mittleren Aktivität. Dies ist derjenige Prozentsatz an Zeit, während der der Empfänger im Mittel angeschaltet ist. Außerdem bestimmt sich der Stromverbrauch nach dem Stromverbrauch im aktiven Betrieb. Die Aktivität berechnet sich aus der Summe aller Paketfrequenzen multipliziert mit der Einschaltdauer, die für den Empfang eines Pakets benötigt wird. Die niedrige Aktivität des erfindungsgemäßen Systems wird dadurch erreicht, daß der Empfänger im belasteten Betrieb zwischen Empfänger und Sender genau weiß, wann er das nächste Fixpaket eines beliebigen Senders aus seinem System zu erwarten hat. Dadurch kann der Empfänger gerade für diejenige Zeitspanne eingeschaltet werden, die für den Empfang eines Pakets benötigt wird. Bei einem aktiven Stromverbrauch im Empfänger von 4 mA und einer Einschaltdauer von 3 msec sowie bei einer Paketfrequenz pro Sender für die fixen Pakete von 1 Hz ergibt sich bei insgesamt vier Sendern eine Aktivität von 1,2%. Daraus resultiert eine Stromaufnahme von 48 µA. Der minimal realisierbare Stromverbrauch hängt dabei noch von der Technologie der verfügbaren Empfängerchips ab.

Die Sender können als Mikrocontroller mit internem EEPROM für die ID-Nummer ausgeführt sein. Die jeweilige Sub-ID und die Paketfrequenz des Systems können durch externe Pins fest einstellbar oder durch Einträge im EEPROM konfigurierbar sein. Die ID wird nach einem Reset aus dem EEPROM eingelesen oder nach dem Zufallsprinzip generiert. Zu Testzwecken wird kurz nach dem Reset die gesamte ID einmal ausgesendet. Beim Einsatz von Mikroprozessoren mit integriertem EEPROM kann die ID beim Test auch seriell in den Baustein geschrieben und durch nochmaliges Reset auf korrektes Schreiben hin überprüft werden.

Das Programm zur Steuerung des Mikrocontrollers arbeitet gemäß dem erfindungsgemäßen Verfahren. Es kann folgendermaßen strukturiert sein. Es gibt insgesamt zwei Interrupts, die von einem Timer bzw. vom Meßwertgeber ausgelöst werden können. Der Timer-Interrupt muß die höhere Priorität besitzen und wird beispielsweise von einem stabilem Quarztakt mit einer Frequenz von 33 kHz abgeleitet. Für das serielle Aussenden der Pakete wird kurzzeitig ein Prozessortakt von ca. 150 kHz bis 300 kHz nötig. Wenn gerade kein Interrupt bedient wird, legt sich der Prozessor schlafen.

Nach einem Reset wird entweder der Test-Modus oder der ID-Search-Modus angesprungen. Der Test-Modus führt Aktionen aus, die einem Fertigungstest dienen können und hält dann an. Auf den ID-Search-Modus, indem eine Zufallszahl als ID des Senders generiert wird, folgt nach dem ersten Meßinterrupt der Aktiv-Modus. Im Aktiv-Modus wird gemessen und übertragen. Der Aktiv-Modus geht in eine Zero-Modus über, wenn über eine kurze Zeit lang keine Meßimpulse mehr registriert wurden. Wenn sichergestellt ist, daß genügend lang "Null" übertragen wurde, wird der Stand-By-Modus aktiviert und die Übertragung abgeschaltet. Der Timerinterrupt läuft dabei weiter. Nach einer zusätzlichen Zeit in der Größenordnung mehrerer Stunden beginnt der Power-Down-Modus. In diesem Fall schaltet der Prozessor den Timerinterrupt ab. Die ID bleibt im Sender erhalten.

Die Routine, die das Datenpaket aussendet, ist zeitkritisch und wird in Assembler geschrieben. Der Meßinterrupt hat eine niedrigere Priorität als der Timerinterrupt. Wenn der Meßinterrupt auftritt, wird der nächste Meßwert ermittelt und das nächste Paket aufbereitet. Wenn das neue Paket fertig ist, wird ein Pointer vom alten auf das neue Paket verlegt. Der Timerinterrupt darf im Stand-By-Modus nicht abgeschaltet werden. Es ist lediglich eine Abfrage nötig, die das Aussenden des Pakets umgeht.

Falls ein Watchdog aktiviert ist, muß dieser zum Beispiel im Timerinterrupt oder sogar auch im Meßinterrupt regelmäßig bedient werden. Im Power-Down-Modus schlägt der Watchdog ständig an, bis irgendwann wieder ein Meßinterrupt auftritt. Dies richtet keinen Schaden an und ist vom Stromverbrauch her unbedenklich.

Die Erfindung ist auch in einem Fahrradcomputer verwirklicht, der wenigstens einen erfindungsgemäßen Sender und einen erfindungsgemäßen Empfänger aufweist. Er kann insbesondere mit einem Sensor zur Messung von Raddrehungen eines Fahrrads, mit einem Sensor zur Messung von Kurbeldrehungen oder mit einem Sensor zur Messung des Fahrtwiderstands des Fahrrads verbunden sein, wobei der Empfänger ferner ein Display zur Anzeige des vom Sensor übertragenen Werts aufweisen kann. Ein solcher Fahrradcomputer läßt sich besonders zuverlässig betreiben, wobei stets genaue Meßergebnisse angezeigt werden.

Schließlich ist es noch denkbar, die erfindungsgemäße Funkstrecke in einem Fitneßcomputer vorzusehen, bei dem ein Sender mit einem Sensor zur Messung des Herzschlags eines Benutzers oder zur Messung dessen Schrittfrequenz verbunden sein kann. Ein solcher Fitneßcomputer kann einen Empfänger mit Display zur Anzeige des vom Sensor übertragenen Werts aufweisen.

Die Erfindung ist in der Zeichnung anhand eines Ausführungsbeispiels näher veranschaulicht.
- Figur 1: zeigt eine schematische Darstellung einer erfindungsgemäßen Funkstrecke, die drei Sender und einen Empfänger aufweist,
- Figur 2: veranschaulicht die von den Sendern aus Figur 1 abgegebenen Signale im Hinblick auf deren zeitliche Abfolge,
- Figur 3: veranschaulicht den Aufbau eines von einem Sender aus Figur 1 abgegebenen Datenpaket oder dessen Duplikat,
- Figur 4: zeigt ein Zustandsdiagramm, das den Betrieb des Empfängers aus Figur 1 veranschaulicht,
- Figur 5: veranschaulicht einen Einrastmodus des Empfängers aus Figur 1 gemäß einer ersten Signalsituation,
- Figur 6: veranschaulicht einen Einrastmodus des Empfängers aus Figur 1 gemäß einer zweiten Signalsituation,
- Figur 7: veranschaulicht einen Betriebsmodus eines Empfängers aus Figur 1 und
- Figur 8: veranschaulicht einen weiteren Betriebsmodus des Empfängers aus Figur 1.

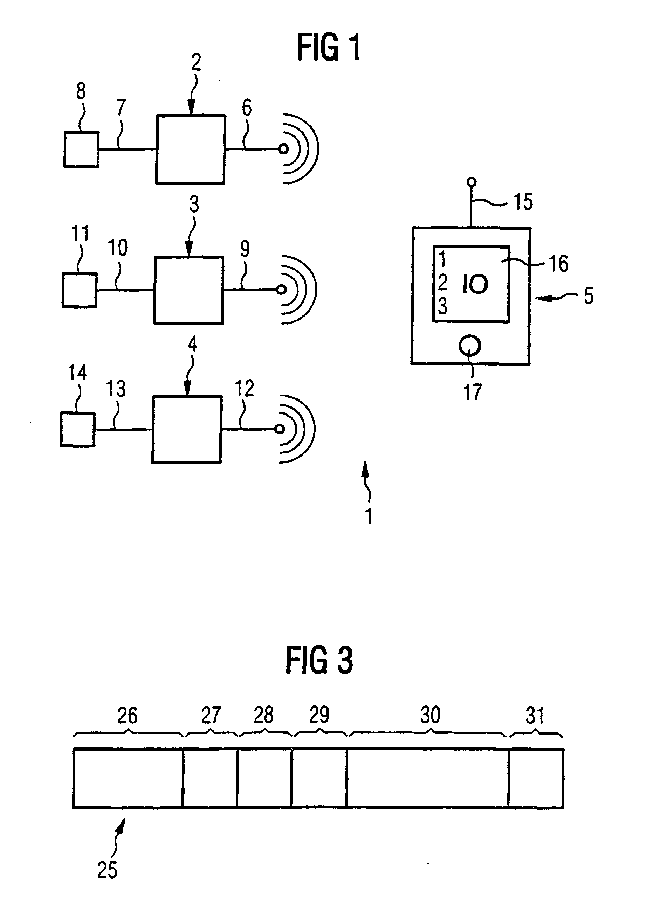

Figur 1 zeigt eine schematische Darstellung einer erfindungsgemäßen Funkstrecke 1, die einen ersten Sender 2, einen zweiten Sender 3, einen dritten Sender 4 sowie einen Empfänger 5 umfaßt. Der erste Sender 2 hat eine erste Antenne 6 zum Ausenden von Funksignalen. Die erste Antenne 6 steht mit einer in dieser Ansicht nicht gezeigten Sendeeinheit in Verbindung, die die von der ersten Antenne 6 auszusendenden Funksignale generiert. Weiterhin umfaßt der erste Sender 2 eine in dieser Ansicht nicht gezeigte Sendersteuereinheit zur Ansteuerung der Sendeeinheit. Die Sendersteuereinheit empfängt über eine erste Eingangsleitung 7 Signale von einem ersten Sensor 8, der als Umdrehungszähler eines in dieser Ansicht nicht gezeigten Laufrades ausgebildet ist. Der erste Sensor 8 liefert somit über die erste Eingangsleitung 7 Lageinformationen über das Laufrad an die Sendersteuereinheit. Die Sendersteuereinheit wandelt diese Lageinformationen in digitale Daten um und veranlaßt die Sendeeinheit, diese digitalen Daten über die erste Antenne 6 auszusenden.

Der zweite Sender 3 hat eine zweite Antenne 9 sowie eine zweite Eingangsleitung 10 und stimmt hinsichtlich seines übrigen Aufbaus im wesentlichen mit dem ersten Sender 2 überein. Der zweite Sender 3 empfängt über die zweite Eingangsleitung 10 Signale eines zweiten Sensors 11, der Lageinformationen über eine hier nicht gezeigte Tretkurbel eines Fahrrads auswertet. Der zweite Sender 3 wandelt diese Lageinformationen in digitale Signale um, die über die zweite Antenne 9 als Funksignale abgegeben werden.

Der dritte Sender 4 umfaßt eine dritte Antenne 12 sowie eine dritte Eingangsleitung 13, über die Daten von einem dritten Sensor 14 aufgenommen werden. Hinsichtlich seines übrigen Aufbaus stimmt der dritte Sender 4 im wesentlichen mit dem ersten Sender 2 überein. Der dritte Sensor 14 bestimmt die Herzschlagsfrequenz eines in dieser Ansicht nicht gezeigten Menschen, der sich auf einem Fahrrad fortbewegt. Diese Herzschlagsfrequenz wird vom dritten Sender 4 in digitale Daten umgewandelt und über die dritte Antenne 12 als Funksignal abgegeben.

Der Empfänger 5 hat eine Empfängerantenne 15 zum Empfang der vom ersten Sender 2, vom zweiten Sender 3 und vom dritten Sender 4 abgegebenen Funksignale. Die von der Empfängerantenne 15 empfangenen Funksignale werden an eine in dieser Ansicht nicht gezeigte Empfangseinheit weitergeleitet, die betätigbar mit einer in dieser Ansicht ebenfalls nicht gezeigten Empfängersteuereinheit verbunden ist. Die Empfangseinheit kann von der Empfängersteuereinheit ein- und ausgeschaltet werden. Die Empfangersteuereinheit kann sich außerdem auch selbst in einen ausgeschalteten Zustand versetzen. Die Empfängersteuereinheit wandelt die von der Empfangseinheit aufgenommenen Daten um und zeigt diese auf einem Display 16 an. Auf dem Display 16 kann dabei angezeigt werden, von welchem Sender die im Display 16 angezeigten Daten ausgesendet wurden. Außerdem können auch die Inhalte der jeweiligen Daten angezeigt werden. Weiterhin weist der Empfänger 5 eine Bedienertaste 17 auf, mit der die Empfängersteuereinheit von einem Benutzer betätigt werden kann.

Im Betrieb der Funkstrecke 1 senden der erste Sender 2, der zweite Sender 3 und der dritte Sender 4 ständig wiederholend Daten aus, die vom Empfänger 5 empfangen, ausgewertet und auf dem Display 16 angezeigt werden.

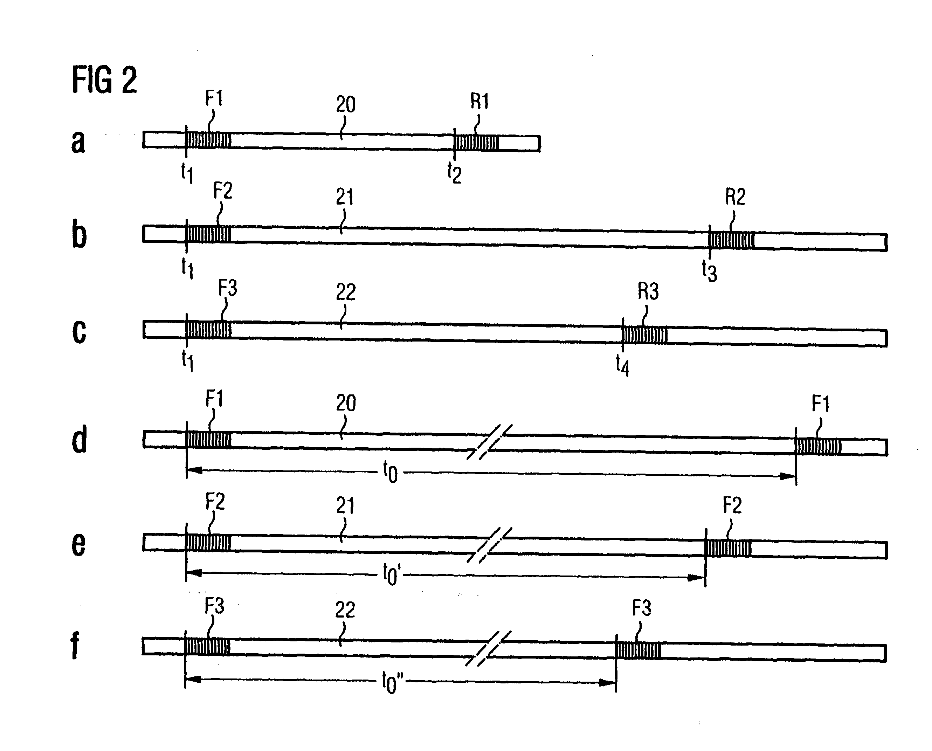

Figur 2 veranschaulicht die vom ersten Sender 2, vom zweiten Sender 3 und vom dritten Sender 4 abgegebenen Funksignale hinsichtlich der zeitlichen Abfolge der durch sie übermittelten Daten.

Figur 2a zeigt einen ersten Zeitstrahl 20, der ein erstes Fixpaket F1 sowie ein erstes Redundanzpaket R1 enthält. Das erste Fixpaket F1 und das erste Redundanzpaket R1 werden durch jeweils ein Datensignal gebildet, die auf ein Trägersignal mit einer Trägersignalfrequenz aufmoduliert sind. Hierfür kann grundsätzlich ein beliebiges Modulationsverfahren verwendet werden.

Wie man in Figur 2a besonders gut sieht, beginnt das Aussenden des ersten Fixpakets F1 zum Zeitpunkt t₁. Das Aussenden des ersten Redundanzpakets R1 beginnt zu einem Zeitpunkt t₂. Die Länge des ersten Fixpakets F1 und die Länge des ersten Redundanzpakets R1 stimmt im wesentlichen überein.

Figur 2b veranschaulicht anhand eines zweiten Zeitstrahls 21 das vom zweiten Sender 3 abgegebene Funksignal. Es umfaßt ein zweites Fixpaket F2 und ein zweites Redundanzpaket R2, die als Datensignale auf eine Trägerfrequenz aufmoduliert sind, die mit der Trägerfrequenz des ersten Senders 2 identisch übereinstimmt. Das zweite Fixpaket F2 wird zu einem Zeitpunkt t₁ ausgesendet und das zweite Redundanzpaket R2 wird zu einem Zeitpunkt t₃ ausgesendet, wobei die Differenz t₃-t₂ beim zweiten Sender 3 kleiner ist als die Differenz t₂-t₁ beim ersten Sender 2. Auf diese Weise wird eine zeitliche Kollision von ersten Fixpaket F1, zweiten Fixpaket F2, ersten Redundanzpaket R1 und zweiten Redundanzpaket R2 in der Weise vermieden, daß zumindest eines der Datenpakete pro Sender nicht mit allen Datenpaketen des anderen Sender zeitlich zusammenfällt.

Figur 2c veranschaulicht einen dritten Zeitstrahl 22, auf dem ein vom dritten Sender 4 ausgesendetes drittes Fixpaket F3 sowie ein drittes Redundanzpaket R3 eingezeichnet sind.

Der dritte Sender 4 sendet auf der gleichen Trägerfrequenz wie der erste Sender 2 und der zweite Sender 3, wobei das dritte Fixpaket F3 und das dritte Redundanzpaket R3 auf ein entsprechendes Trägersignal aufmoduliert sind. Wie man in Figur 2c besonders gut sieht, beginnt das Aussenden des Fixpakets F3 zum Zeitpunkt t₁, während das Aussenden des dritten Redundanzpakets zum Zeitpunkt t₄ beginnt. Dabei unterscheidet sich die Zeitdifferenz t₄-t₁ des dritten Senders 4 von den entsprechenden Zeitdifferenzen t₃-t₁ des zweiten Senders 3 und t₂-t₁ des ersten Senders 2.

Figur 2d veranschaulicht anhand des ersten Zeitstrahls 20 den zeitlichen Abstand t₀ zwischen zwei Fixpakten F1 des ersten Senders 2. Der Abstand t₀ ist zwischen je zwei aufeinanderfolgenden Fixpaketen F1 im wesentlichen identisch. Demzufolge werden die Fixpakete F1 in regelmäßigen zeitlichen Abständen ausgesendet.

Figur 2e veranschaulicht anhand des zweiten Zeitstrahls 21 den zeitlichen Abstand t₀' zwischen je zwei zweiten Fixpaketen F2 des zweiten Senders 3. Die zweiten Fixpakete F2 werden in regelmäßigen zeitlichen Abständen vom zweiten Sender 3 ausgesendet.

Figur 2f veranschaulicht anhand des dritten Zeitstrahls 22 die vom dritten Sender 4 abgegebenen dritten Fixpakete F3. Die dritten Fixpakete F3 werden jeweils mit einem zeitlichen Abstand t₀'' regelmäßig aufeinanderfolgend ausgesendet.

Die jeweiligen zeitlichen Abstände t₀, t₀' und t₀" unterscheiden sich voneinander, so daß sich in den meisten Fällen die vom ersten Sender 2, vom zweiten Sender 3 oder vom dritten Sender 4 ausgesendeten Fixpakete zeitlich nicht überschneiden. Dadurch wird der Betrieb von mehreren Sendern zusammen mit einem einzigen Empfänger ermöglicht.

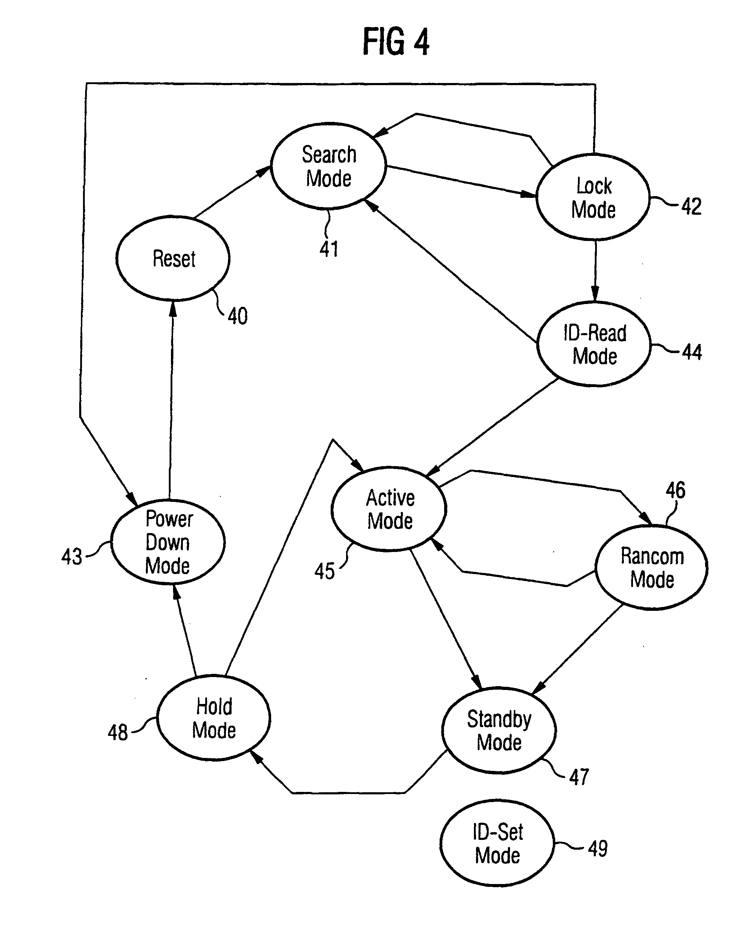

Figur 3 veranschaulicht den Aufbau eines Datenpakets 25, das hinsichtlich seines Aufbaus im wesentlichen mit demjenigen des ersten Fixpakets F1, des zweiten Fixpakets F2, des dritten Fixpakets F3 bzw. mit dem ersten Redundanzpaket R1, mit dem zweiten Redundanzpaket R2 oder mit dem dritten Redundanzpaket R3 übereinstimmt. Das Datenpaket 25 gliedert sich in einen ersten Identifikationsbereich 26, in einen Typbereich 27, in einen Versetzungsinformationsbereich 28, in einen zweiten Identifikationsbereich 29, in einen Nutzdatenbereich 30 sowie in einen Prüfcodebereich 31.

Dabei dient der erste Identifikationsbereich 26 zur Aufnahme von Informationen, die die sogenannte Sub-ID des jeweiligen Senders angeben. Die Sub-ID des jeweiligen Senders kann eine Information über die zeitliche Versetzung zwischen einem von dem Sender ausgesendeten Fixpaket und dem entsprechenden Redundanzpaket aufnehmen. Ähnlich der Vergabe von Kanälen bei Funkstrecken, die vorgegebene Bandbreiten von Funksignalen ausnützen, können so Zeitscheibenbereiche bei der Nutzung einer einzigen vorgegebenen Übertragungsfrequenz ausgenutzt werden. Die so definierten Zeitscheibenbereiche werden vorteilhafterweise eindeutig gekennzeichnet und - jeweils einem Sender zugeordnet - im ersten Identifikationsbereich eines vom betreffenden Sender ausgesendeten Fixpakets bzw. Redundanzpakets wiedergegeben.

Der Typbereich 27 nimmt Informationen darüber auf, ob es sich bei dem jeweiligen Datenpaket um ein Fixpaket oder um ein Redundanzpaket handelt.

Der Versetzungsinformationsbereich 28 bleibt bei einem einfachen Betrieb der erfindungsgemäßen Funkstrecke 1 ohne Redundanzpakete ungenutzt. In einer vorteilhaften Weiterbildung geben die im Versetzungsinformationsbereich 28 enthaltenen Informationen zusammen mit den Informationen im ersten Identifikationsbereich 26 darüber Auskunft, in welchem zeitlichen Abstand das einem Fixpaket folgende Redundanzpaket erscheint. Hierzu kann in dem entsprechenden Sender des Datenpakets 25 eine Rechenregel bzw. eine Zählfolge vorgesehen sein, gemäß der der zeitliche Abstand zwischen Fixpaket und dem daraufhin ausgesendeten Redundanzpaket festgelegt ist. Wenn eine solche Folge über einen Zähler gesteuert wird, dann kann der jeweilige Zählerstand in dem Versetzungsinformationsbereich 28 festgehalten und ausgesendet werden.

Der zweite Identifikationsbereich 29 kann zur Aufnahme einer jeweils nur einmal vergebenen Seriennummer verwendet werden, durch die sich eine Unterscheidung eines Senders der Funkstrecke 1 von einem Sender einer anderen Funkstrecke ermöglichen läßt.

Der Nutzdatenbereich 30 nimmt die durch die Funkstrecke 1 zu übertragenden Daten auf.

Der Prüfcodebereich 31 dient bei einem Empfang des Datenpakets 25 zur Feststellung , ob die übertragenen Daten in der Zwischenzeit unerwünschte Veränderungen erfahren haben. Zur Generierung des Inhalts des Prüfcodebereichs 31 kann beispielsweise ein Paritätsverfahren oder ein Prüfsummenverfahren verwendet werden.

Weiterhin weist das Datenpaket 25 noch in dieser Ansicht nicht gezeigte Scan, -Synchronisations- und Startdaten auf, die insbesondere durch die maschinelle Verarbeitung des Datenpakets 25 bedingt und entsprechend ausgebildet sind.

Figur 4 zeigt ein Zustandsdiagramm, das das Verhalten des Empfängers 5 aus Figur 1 in seinen verschiedenen Betriebsmodi beschreibt. Dabei werden insbesondere die Übergänge zwischen dessen einzelnen Betriebsmodi veranschaulicht.

In einem Ausgangszustand des Empfängers 5 wird dieser durch einen Druck auf eine in Figur 1 nicht gezeigte Reset-Taste in einen Reset-Modus 40 versetzt. In dem Reset-Modus 40 werden die Software- und Hardwaremodule des Empfängers 5 initialisiert. Ausgehend vom Reset-Modus 40 geht der Empfänger 5 nachfolgend in einen Such-Modus 41 über, wenn ein normaler Betrieb des Empfängers 5 erfolgen soll.

Im Such-Modus 41 wird das Zeitraster für die Fixpakete F1, F2 und F3 des ersten Senders 2, des zweiten Senders 3 und des dritten Senders 4 innerhalb der Funkstrecke 1 ermittelt. Hierzu wird eine Liste mit einzelnen Zeitpunkten für den Eingang jeweils eines Fixpakets eines Senders aufgestellt. Der Empfänger 5 tastet dabei mit vielen kurzen Aktivitäten einen Zeitraum ab, der einem maximalen zeitlichen Abstand zwischen zwei Fixpaketen eines in der Funkstrecke 1 enthaltenden Senders entspricht. Dabei werden die Aktivitäten auf der Funkstrecke 1 erfaßt. In den meisten Fällen wird innerhalb dieses Zeitraums von jedem Sender genau ein Fixpaket abgetastet werden. Die jeweilige Dauer der einzelnen Abtastungen soll größer sein als die maximal vorkommende sendeaktivitätsfreie Zeit innerhalb eines Datenpakets. Der Abstand der einzelnen Abtastungen wird vorzugsweise etwas kleiner als eine Paketlänge gehalten. Aus diesen Abtastungen ergibt sich eine Liste von Zeiten, zu denen Aktivitäten der Funkstrecke 1 festgestellt worden sind. Diese Aktivitäten können sowohl Fixpakete als auch Redundanzpakete umfassen und sowohl von der Funkstrecke 1 als auch weiteren, in Figur 1 nicht dargestellten Funkstrecken stammen. Darüber hinaus können auch Funkstörungen abgetastet werden.

In einer in Figur 4 nicht dargestellten Variante kann ausgehend vom Reset-Modus 40 auch in einen Test-Modus verzweigt werden, der für einen speziellen Betrieb des Empfängers 5 im Anschluß an dessen Fertigung bestimmt ist. Im Test-Modus können einzelne Funktionen des Empfängers 5 getestet werden. Hierzu wird ein spezieller Betrieb des Empfängers 5 hergestellt. Ausgehend vom Test-Modus kann nur in den Reset-Modus 40 zurück verzweigt werden können.

Ausgehend vom Such-Modus 41 geht der Empfänger 5 in einen Einrast-Modus 42 über, in dem Fixpakete jeweils des ersten Senders 2, des zweiten Sender 3 bzw. des dritten Senders 4 zu den im Such-Modus 41 vorausberechneten Zeitpunkten gesucht werden. Zum Vorausberechnen der Zeitpunkte des Eingangs je eines Fixpaketes der in der Funkstrecke 1 vorhandenen Senders wird dabei ausgenutzt, daß die im Such-Modus 41 empfangenen Fixpakete im jeweiligen ersten Identifikationsbereich 26 eine eindeutige Information darüber enthalten, in welchem regelmäßigen zeitlichen Abstand die Fixpakete je eines Senders ausgesendet werden. Damit läßt sich nach dem Empfangen eines von einem Sender ausgesendeten Fixpaketes auf einfache Weise der Eingang des nächsten Fixpaketes vorhersagen. Somit ist der Empfänger 5 in der Lage, sich auf die Fixpakete des jeweiligen Senders zu synchronisieren, sobald je ein Fixpaket passend zur gesuchten Sub-ID korrekt empfangen wurde.

Ausgehend vom Einrast-Modus 42 kann sich der Empfänger 5 wieder in den Such-Modus zurückversetzen, wenn im Such-Modus 41 kein Fixpaket einer gesuchten Sub-ID aufgefunden wurde.

Schließlich kann aus dem Einrast-Modus 42 auch in einen sogenannten Ausschalt-Modus 43 verzweigt werden, wenn zu viele hintereinander erfolgende Wechsel zwischen Einrast-Modus 42 und dem Such-Modus 41 erfolgt sind. In einem solchen Fall ist kein ordnungsgemäßer Betrieb der Funkstrecke 1 möglich, so daß sowohl die Empfangseinheit als auch die Empfängersteuereinheit vollständig ausgeschaltet werden können. Aus dem Ausschalt-Modus 43 kann der Empfänger 5 durch Betätigen der Reset-Taste in Reset-Modus 40 versetzt werden.

Ausgehend vom Einrast-Modus 42 versetzt sich der Empfänger 5 in einen ID-Such-Modus 44, in dem auf Fixpakete der Sender der Funkstrecke 1 gewartet wird, in denen der jeweilige Versetzungsinformationsbereich 28 einen vorbestimmten Inhalt aufweist. Mit dem vorbestimmten Inhalt des Versetzungsinformationsbereichs 28 kann ein in Figur 1 nicht gezeigter Zahlensequenzgenerator des Empfängers 5 gestartet werden, der gemäß einer Rechenregel zur Berechnung der jeweiligen Versetzung zwischen Fixpaket und Redundanzpaket eines Senders verwendet werden kann. Außerdem kann beim Auftreten eines vorbestimmten Inhalts im Versetzungsinformationsbereich 28 begonnen werden, über mehrere Datenpakete hinweg übertragene, zusammengehörende Inhalte des jeweiligen zweiten Identifikationsbereichs 29 zu einer Gesamtinformation zusammenzusetzen. Dabei gibt der Inhalt des Versetzungsinformationsbereichs 28 auch eine Information darüber, wie diese aufeinanderfolgenden Informationen der jeweiligen zweiten Identifikationsbereiche 29 zusammenzusetzen sind.

Für den Fall, daß die während des ID-Such-Modus empfangenen IDs nicht mit den erwarteten IDs des ersten Senders 2, des zweiten Senders 3 und des dritten Senders 4 übereinstimmen, geht der Empfänger 5 in den Such-Modus 41 zurück.

Im Normalfall, in dem das Vorhandensein des ersten Sender 2, des zweiten Senders 3 und des dritten Senders 4 abgetastet wird, geht der Empfänger 5 in einen Aktiv-Modus 45 über. Im Aktiv-Modus 45, der den Normalbetrieb der Funkstrecke 1 repräsentiert, wird die Empfangseinheit im wesentlichen ausgeschaltet gehalten. Lediglich zu Zeitpunkten, an denen die Empfängersteuereinheit den zu erwartenden Eingang eines Fixpakets eines der Sender erwartet, wird die Empfangseinheit eingeschaltet. Im Aktiv-Modus 45 wird die genaue Lage der empfangenen Fixpakete ständig überprüft und gegebenenfalls kleinere Fehler in der Lageberechnung durch die Empfängersteuereinheit korrigiert. Im Aktiv-Modus 45 werden außerdem die Daten innerhalb der Funkstrecke 1 übertragen, von der Empfängersteuereinheit ausgewertet und an das Display 16 weitergeleitet.

Ausgehend vom Aktiv-Modus 45 begibt sich der Empfänger 5 in einen Redundanz-Modus 46, wenn die im Prüfcodebereich 31 eines empfangenen Fixpakets enthaltene Information bei der Auswertung des Datenpakets daraufhin deutet, daß das Datenpaket verfälscht oder unvollständig übertragen worden. Im Redundanz-Modus 46 wird die Empfangseinheit von der Empfängersteuereinheit dann eingeschaltet, wenn zu dem falsch oder nicht empfangenen Fixpaket zu einer bestimmten ID eines der Sender ein Redundanzpaket auszuwerten ist. Die genaue zeitliche Lage des Empfangs des Redundanzpakets zu dem betreffenden Fixpaket ergibt sich aus der in der Empfängersteuereinheit weiterverfolgten Zahlenfolge.

Nach dem Empfangen des entsprechenden Redundanzpakets geht der Empfänger 5 wieder in den Aktiv-Modus 45 zurück, in dem er aufeinanderfolgende Fixpakete auswertet.

Ausgehend vom Aktiv-Modus 45 oder vom Redundanz-Modus 46 geht der Empfänger 5 in einen Standby-Modus 47 über, wenn für eine vorbestimmte Zeit von beispielsweise 3 Sekunden weder Fixpakete noch Redundanzpakete empfangen werden. In diesem Zustand wird die Empfangseinheit mit einer herabgesetzten Häufigkeit eingeschaltet und auf das Vorhandensein von Fixpaketen oder Redundanzpaketen hin untersucht. Dadurch wird ein verringerter Stromverbrauch gewährleistet, wenn die Funkstrecke 1 beispielsweise durch eine zu große Entfernung zwischen dem Empfänger 5 und den Sendern unterbrochen ist. Bei einer Annäherung des Empfängers 5 an die Sender wird der normale Betrieb der Funkstrecke im Aktiv-Modus 45 bzw. im Redundanz-Modus 46 wieder aufgenommen.

Ausgehend vom Standby-Modus 47 geht der Empfänger 5 in einen Hold-Modus 48 über, wenn ein bestimmter Zeitraum seit dem letzten Empfangen eines Fixpakets oder eines Redundanzpakets vergangen ist. Im Hold-Modus 48 bleibt die Empfangseinheit solange abgeschaltet, bis die Bedienertaste 17 betätigt wird. In diesem Fall versetzt sich der Empfänger 5 in den Aktiv-Modus 45, in dem sofort Fixpakete zu den vorbestimmten Zeitpunkten empfangen werden können, weil die Empfängersteuereinheit die zu erwarteten Zeitpunkte für den Eingang von Fixpaketen weiter berechnet hat. Wenn in diesem Zustand aus dem Aktiv-Modus 45 in den Redundanz-Modus 46 verzweigt wird, können auch Redundanzpakete unmittelbar eingelesen werden, weil die Empfängersteuereinheit die Zahlenfolge für die Zeitpunkte für den zu erwartenden Eingang je eines Redundanzpakets ebenfalls weiter berechnet hat.

Falls die Bedienertaste 17 des Empfängers 5 nicht betätigt worden ist, wenn sich dieser im Standby-Modus befindet, begibt sich der Empfänger 5 aus dem Hold-Modus 48 automatisch in den Ausschalt-Modus 43, wenn er sich für einen bestimmten Zeitraum im Hold-Modus 48 befunden hat. Ein solcher Übergang vom Hold-Modus 48 in den Ausschalt-Modus 43 ist auch dann denkbar, wenn die Empfängersteuereinheit zu große Toleranzen für die weiterberechneten Eingangszeiten von Fixpaketen oder Redundanzpaketen feststellt.

Eine besondere Situation für den Empfänger 5 stellt ein Anlern-Modus 49 dar, in dem die Funkstrecke 1 abgeschirmt von anderen Funkstrecken und Störungen im Such-Modus 41, im Einrast-Modus 42 und im ID-Modus 44 betrieben wird. Zusätzlich zu dem Verhalten in den vorstehend aufgeführten Modi wird im Anlern-Modus 49 abgetastet, welche Sender sich in der Umgebung des Empfängers 5 befinden. Deren IDs werden dann empfangen und dauerhaft im Empfänger 5 abgelegt. Dabei muß selbstverständlich gewährleistet sein, daß alle Sender der Funkstrecke 1 aktiv sind und daß keine Sender einer zweiten Funkstrecke die Übertragung stören. Der Anlern-Modus 49 wird durch Betätigen einer in Figur 1 nicht gezeigten Anlerntaste des Empfängers 5 erreicht und wieder verlassen.

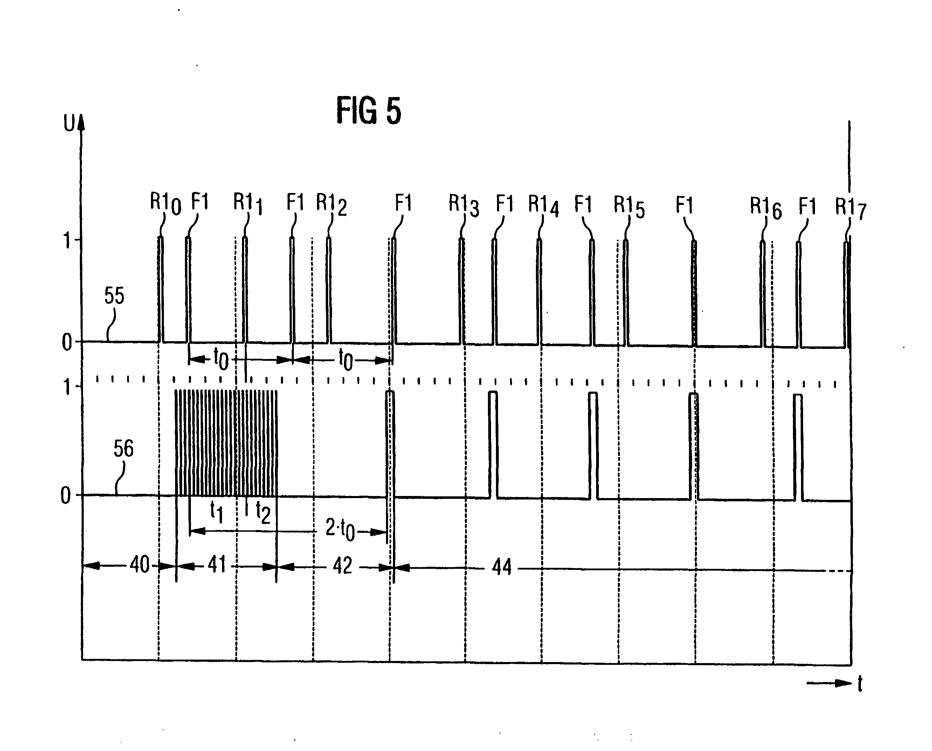

Figur 5 zeigt ein Einschaltdiagramm, das die Einschaltzeiten des ersten Senders 2 und des Empfängers 5 veranschaulicht. In der folgenden Beschreibung der Erfindung wird ausgenommen, daß nur der erste Sender 2 aktiv ist. Der zweite Sender 3 und der dritte Sender 4 sind inaktiv. Dabei gibt die im oberen Teil des Einschaltdiagramms wiedergegebene Senderkurve 55 an, wann die Sendeeinheit eingeschaltet ist. Zu diesen Zeitpunkten befindet sich der Pegel der Senderkurve 55 auf dem Wert "1". Zu Zeitpunkten, an denen die Sendeeinheit ausgeschaltet ist, nimmt die Senderkurve 55 den Wert "0" an. Beim Auftreten eines Fixpakets bzw. eines Redundanzpakets, das vom ersten Sender 2 ausgeht, erscheint deshalb ein Peak in der Sendekurve 55. Oberhalb der jeweiligen Peaks der Senderkurve 55 sind die Bezeichnungen der ersten Fixpakete F1 bzw. der ersten Redundanzpakete R1 angegeben. Insgesamt weist die Senderkurve 55 acht Redundanzpakete R1 auf, deren Index von "0" bis "7" durchnumeriert ist. Außerdem sind insgesamt sieben Fixpakete F1 vorgesehen, die zeitlich äquidistant zueinander angeordnet sind.

Der zeitliche Abstand zwischen je einem Fixpaket F1 und dem zugeordneten Redundanzpaket R1 ergibt sich aus einem Zählerstand, der im ersten Sender 2 gemäß einer festgelegten Zahlenfolge fortgeführt wird. Wie man in Figur 5 besonders gut sieht, variiert der zeitliche Abstand zwischen jedem Fixpaket F1 und dessen zugeordnetem Redundanzpaket R1.

Unterhalb der Senderkurve 55 ist eine Empfängerkurve 56 dargestellt, die den Einschaltzustand der Empfangseinheit anzeigt. Der Einschaltzustand der Empfangseinheit wird dabei mit "1" bezeichnet, wenn sich die Empfangseinheit in eingeschaltetem Zustand befindet, und mit "0" bezeichnet, wenn sie sich in ausgeschaltetem Zustand befindet.

Unterhalb der Empfängerkurve 56 werden die einzelnen Zustände des Empfängers 5 nach Aufnahme der Tätigkeit der Funkstrecke 1 bezeichnet. Nach einem Betätigen der Reset-Taste des Empfängers 5 geht dieser in den Reset-Modus 40 über, in dem die Empfangseinheit ausgeschaltet ist. Daraufhin geht der Empfänger 5 in den Such-Modus 41 über. Im Such-Modus 41 wird die Empfangseinheit in regelmäßigen Abständen jeweils für einen kurzen Zeitraum eingeschaltet, der kürzer ist als die übertragungszeit eines Datenpakets. Während des Such-Modus 41 können durch die Aktivitäten des Empfängers 5 somit noch keine Auswertungen von einzelnen Datenpaketen erfolgen, sondern lediglich Informationen darüber erhalten werden, ob in der Funkstrecke 1 irgendweiche Sendeaktivitäten der beteiligten Sender zu verzeichnen sind.

Kurz nach dem Übergang vom Reset-Modus 40 in den Such-Modus 41 nimmt der Empfänger 5 ein Fixpaket F1 wahr, das gemäß dem Diagramm in Figur 2 zum Zeitpunkt t₁ ausgesendet ist. Kurz nach dem Eingang des Fixpakets F1 nimmt der Empfänger 5 das Redundanzpaket Rl₁ wahr, und zwar zum Zeitpunkt t₂ gemäß dem Diagramm aus Figur 2. Dem Empfänger 5 ist bekannt, daß eine der Wiederholzeiten für Fixpakete in der Funkstrecke 1 gleich t₀ als dem Abstand zwischen zwei Fixpaketen F1 des ersten Senders 2 ist. Dementsprechend geht der Empfänger 5 vom Such-Modus 41 in den Einrast-Modus 42 über und schaltet die Empfangseinheit zum Zeitpunkt 2t₀ für den Empfang eines Datenpakets ein. Zu diesem Zeitpunkt ist wieder ein Fixpaket F1 in der Funkstrecke 1 wahrnehmbar, wie in Figur 5 zu sehen ist. Die Empfangseinheit nimmt das Fixpaket F1 wahr und wertet dieses aus. Somit kann der Empfänger 5 ab diesem Zeitpunkt in den ID-Such-Modus übergehen, in dem die aufeinanderfolgenden Fixpakete F1 mit jeweils gleichbleibendem zeitlichen Abstand t₀ eingelesen und ausgewertet werden.

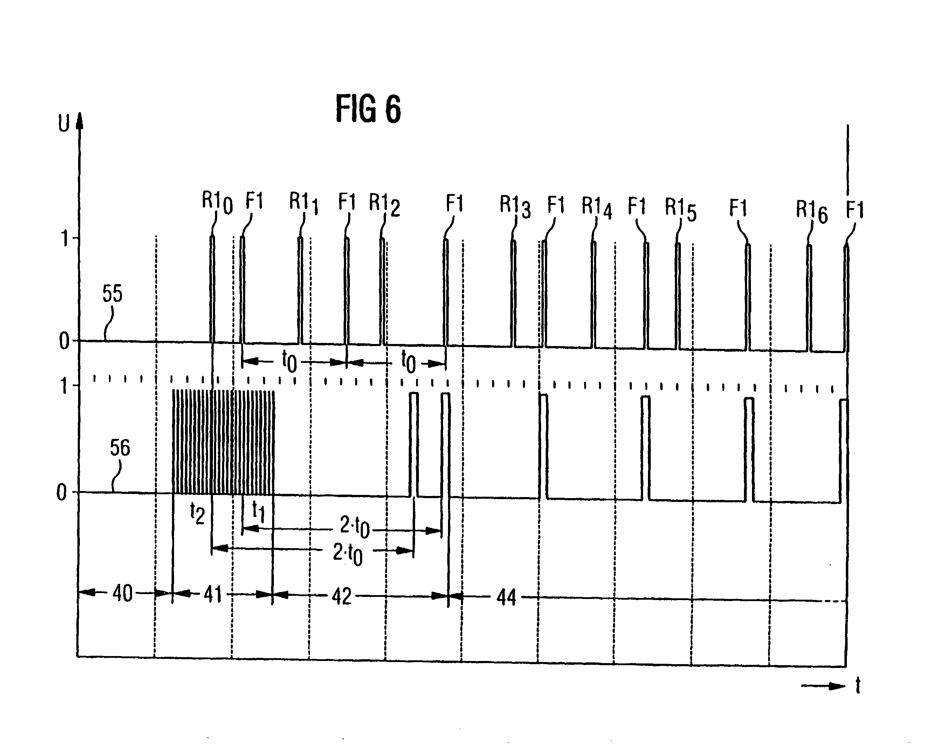

Figur 6 zeigt einen weiteren Einrastvorgang bei der Funkstrekke 1, der in wesentlichen Teilen mit dem Einrastorgang aus Figur 5 übereinstimmt. Gleiche Bestandteile haben deshalb die selben Bezugsziffern. Im Unterschied zum Einrastvorgang aus Figur 1 wird während des Such-Modus 41 jedoch zuerst ein Redundanzpaket R1₀ abgetastet, worauf ein Fixpaket F1 folgt. Aufgrund der kurzen Einschaltdauer der Empfangseinrichtung des Empfängers 5 werden nicht jeweils die kompletten Datenpakete empfangen, sondern es wird lediglich jeweils eine Aktivität in der Funkstrecke 1 erfaßt. Die Empfängersteuerungseinheit geht zunächst davon aus, daß es sich bei der ersten abgetasteten Aktivität um ein Fixpaket handelt und versucht, zum Zeitpunkt 2t₀ ein vollständiges Datenpaket als weiteres Fixpaket einzulesen. In Figur 6 wird deutlich, daß diesem versuchten Einlesevorgang eines Datenpakets kein vom ersten Sender 2 ausgesendetes Datenpaket gegenübersteht, so daß hier keine Daten empfangen werden können. Daraus schließt die Empfangseinheit, daß es sich bei der ersten Aktivität um ein Redundanzpaket gehandelt haben muß. Daraus folgt weiterhin, daß es sich bei der zweiten im Such-Modus 41 abgetasteten Aktivität um ein Fixpaket handeln muß. Dementsprechend schaltet die Empfängersteuereinheit die Empfangseinheit zu einem Zeitpunkt 2t₀ nach dem Abtasten dieser zweiten Aktivität erneut ein. Zu diesem Zeitpunkt kann ein Fixpaket empfangen werden, wie in Figur 6 zu sehen ist. Ab diesem Zeitpunkt kann der Empfänger 5 in den ID-Such-Modus umschalten und die weiteren Fixpakete auswerten.

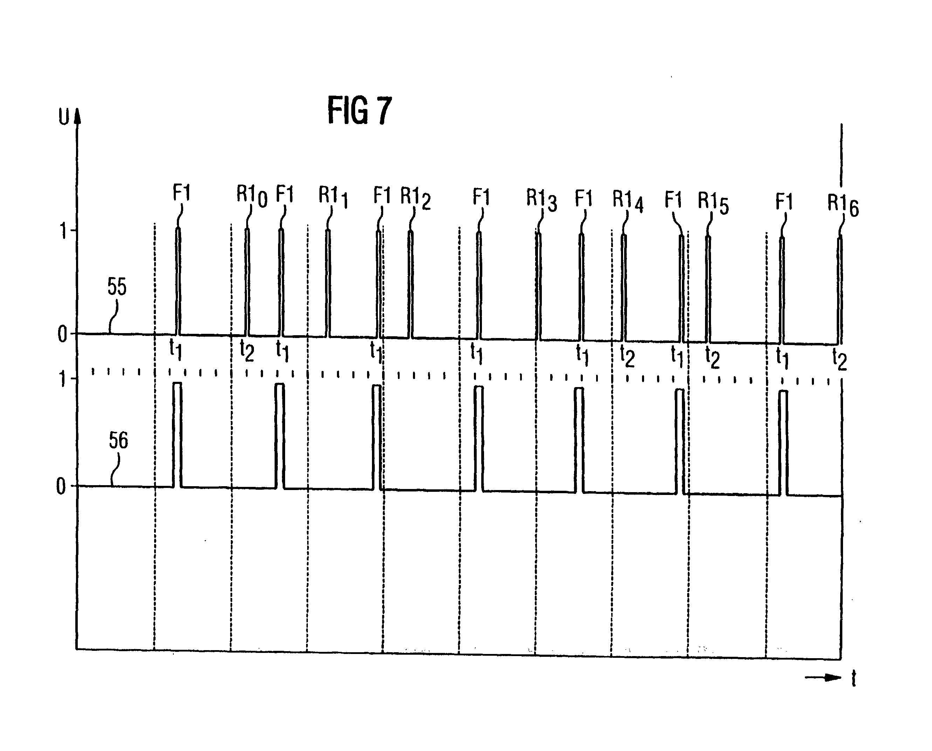

Figur 7 zeigt ein Sende/Empfangsdiagramm, das die Betriebsweise des Empfängers 5 im Aktiv-Modus 45 veranschaulicht. In diesem Zustand ist der Empfänger 5 auf den ersten Sender 2 eingerastet, so daß zu je einem Zeitpunkt t₁ die Empfangseinheit eingeschaltet und das zu diesem Zeitpunkt gesendete Fixpaket F1 empfangen und ausgewertet wird. Hierzu mißt die Empfängersteuereinheit zu jedem Empfangszeitpunkt t₁ die Zeit für den nächsten Empfangszeitpunkt t₁ ab und schaltet die Empfangseinheit entsprechend aus und wieder ein.

In diesem Zustand könnte die Funkstrecke 1 auch betrieben werden, ohne daß der erstes Sender 2 Redundanzpakete aussendet, was ebenfalls Gegenstand der Erfindung ist. Dabei vereinfacht sich auch der in Figur 5 und Figur 6 dargestellte Einrastvorgang.

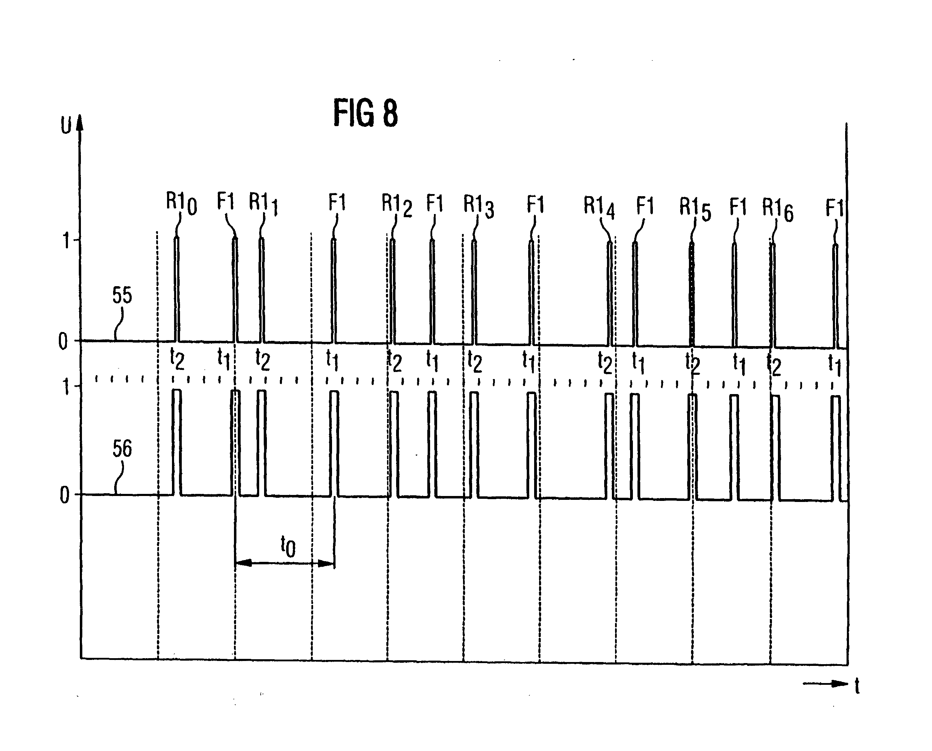

Figur 8 zeigt ein Sende/Empfangsdiagramm bei einem Betrieb des Empfängers 5 im Redundanz-Modus 46. Im Redundanz-Modus 46 werden sowohl die Fixpakete F1 als auch die Redundanzpakete R1₀ bis R1₆ abgetastet. Wie man in Figur 8 besonders gut sieht, variiert dabei der zeitliche Abstand zwischen je einem Fixpaket F1 und einem dazugehörigen Redundanzpaket R1.

Der Empfänger 5 und der erste Sender 2 befinden sich in eingerastetem Zustand, so daß der Empfänger 5 bereits weiß, wann mit dem Eingang von Fixpaketen F1 und Redundanzpakenten R1 zu rechnen ist. Der Eingang der Fixpakete F1 läßt sich errechnen, wenn einmal ein Eingangszeitpunkt eines Fixpakets F1 bekannt ist, indem der unveränderliche zeitliche Abstand t₀ zwischen zwei Fixpaketen F1 zu dem Zeitpunkt des Empfangs des letzten Fixpakets F1 hinzu addiert wird.

Der zeitliche Abstand zwischen einem Fixpaket F1 und dem darauffolgenden Redundanzpaket R1 ergibt sich aus einer Versetzungsregel nach einem Zählerstand im ersten Sender 2. Hierzu wird im ersten Sender 2 bei jedem Aussenden eines Fixpakets ein Zähler um einen bestimmten Wert hochgezählt. Anhand des Zählerstands ergibt sich über eine vorgegebene Reihenfunktion als Versetzungsregel unter Einbeziehung der Sub-ID des ersten Senders 2 sowie unter Einbeziehung weitere Systemparameter ein Zeitpunkt t2 als Zeitverschiebung gegenüber dem Zeitpunkt t1, wobei der Zeitpunkt t2 größer als 0 und kleiner als t₀ ist.

Dem Empfänger 5 wurde zu der betreffenden Sub-ID des ersten Senders 2 ein synchroner Zählerstand mitgeteilt. Der Empfänger 5 verfügt somit über dieselbe Versetzungsregel wie der erste Sender 2 und auch über alle anderen Informationen, die der erste Sender 2 zum Generieren der Sendezeitpunkte für die Redundanzpakete verwendet hat. Durch fortlaufendes Weiterzählen der Zeitverschiebungen für Fixpakete kann der Empfänger 5 somit die zeitliche Lage eines jeden Redundanzpakets R1 vorhersagen und bei Bedarf die Empfangseinheit einschalten. Ein solcher Bedarf ist beispielsweise dann gegeben, wenn ein defektes Fixpaket F1 empfangen wird, so daß die Information aus dem darauffolgenden Redundanzpaket R1 benötigt wird.

## Patentansprüche

1. Sender (2; 3; 4) für eine Funkstrecke (1) mit wenigstens einem Sender und mit wenigstens einem Empfänger, bei der ein aus Datenpaketen (F1; F2; F3) bestehender Datenstrom übertragbar ist,
wobei der Sender (2; 3; 4) die folgenden Merkmale aufweist:
- eine Eingangseinheit zur Eingabe je eines Datenpakets,
- eine Sendersteuereinheit zur Verarbeitung des Datenpakets und zum Einfügen einer sich aus dem Inhalt des Datenpakets ergebenden vorbestimmten Prüfcodierung in das Datenpaket, und
- eine von der Sendersteuereinheit betätigbare Sendeeinheit (6; 9; 12) zur Aussendung des Datenpakets,
wobei der Sender (2; 3; 4) so ausgebildet ist, daß er die Datenpakete (F1; F2; F3) regelmäßig hintereinander aussendet, wobei
die Sendersteuereinheit so ausgebildet ist, daß sie wenigstens ein Duplikat (R1; R2; R3) des Datenpakets erstellt, wobei das Datenpaket (F1; F2; F3) und/oder das Duplikat (R1; R2; R3) jeweils eine Typinformation aufweisen und wobei der Sender (2; 3; 4) so ausgebildet ist, daß er je ein Datenpaket (F1; F2; F3) sowie wenigstens ein Duplikat (R1; R2; R3) regelmäßig hintereinander aussendet und die zeitliche Versetzung zwischen je einem Datenpaket (F1; F2; F3) und dessen Duplikat (R1; R2; R3) gemäß einer vorbestimmten Versetzungsregel und einer in wenigstens einem Datenpaket (F1; F2; F3) und/oder in wenigstens einem Duplikat vorgesehenen Versetzungsinformation variiert, wobei der Sender ferner so ausgestaltet ist, daß er die Versetzungsinformation aus einer vorgegebenen Zählfolge generiert, und zwar unter Verwendung eines Zählerstands der Zählung der vom Sender (2; 3; 4) gesendeten Datenpakete.

2. Sender nach einem der vorhergehenden Ansprüche,
**dadurch gekennzeichnet, daß**
der Sender so ausgestaltet ist, daß er je ein Datenpaket (F1; F2; F3) bzw. dessen Duplikat (R1; R2; R3) mit einer dem Sender (1, 2, 3) zugeordneten Identitätsinformation versieht.

3. Sender nach Anspruch 2,
**dadurch gekennzeichnet, daß**
die Identitätsinformation eine Information über die Art und/oder die Bedeutung der Datenpakete und/oder zumindest einen Teil einer eindeutigen Information über den Sender aufweist.

4. Sender nach einem der vorhergehenden Ansprüche,
**dadurch gekennzeichnet, daß**
die Sendeeinheit (6; 9; 12) von der Sendersteuereinheit zwischen einem eingeschalteten Zustand und einem Zustand mit verringertem Energieverbrauch umschaltbar ist,
und wobei die Sendeeinheit (6; 9; 12) so ausgebildet ist, daß sie selektiv zu Zeitpunkten, an denen Datenpakete (F1; F2; F3) oder Duplikate (R1; R2; R3) gesendet werden, in eingeschaltetem Zustand gehalten ist, und zu Zeitpunkten, an denen keine Datenpakete (F1; F2; F3) bzw. Duplikate (R1; R2; R3) gesendet werden, in dem Zustand mit verringertem Energieverbrauch gehalten ist.

5. Empfänger (5) für eine Funkstrecke (1) mit wenigstens einem Sender (1, 2, 3), insbesondere nach einem der Ansprüche 1 bis 4, bei der ein aus Datenpaketen (F1; F2; F3) und Duplikaten (R1; R2; R3) der Datenpakete bestehender Datenstrom übertragbar ist,
wobei der Empfänger (5) die folgenden Merkmale aufweist:
- eine Empfangseinheit (15) zum Empfang von Datenpaketen (F1; F2; F3),
- eine mit der Empfangseinheit (15) verbundene Empfängersteuereinheit, mit der die empfangenen Datenpakete (F1; F2; F3) verarbeitbar und ausgebbar sind,
wobei die Empfängersteuereinheit so ausgebildet ist, daß
- sie je eine Prüfcodierung eines Datenpakets (F1; F2; F3) ermittelt und aus einem Vergleich der Prüfcodierung mit dem Inhalt des Datenpakets (F1 ; F2; F3) bestimmt, ob ein fehlerfreies oder ein fehlerbehaftetes empfangenes Datenpaket (F1; F2; F3) vorliegt, wobei sie bei einem Empfang eines fehlerbehafteten Datenpakets das betreffende Datenpaket verwirft,
- sie Zeitpunkte für einen zu erwartenden Eingang je eines Datenpakets (F1; F2; F3) bestimmt,
- sie unter Verwendung eines zeitlichen Abstands zwischen zwei Datenpaketen aufeinanderfolgende Datenpakete selektiv auswertet,
wobei die Empfangseinheit (15) so ausgebildet ist, daß Duplikate (R1; R2; R3) der Datenpakete (F1; F2; F3) empfangbar sind und daß die Empfängersteuereinheit so ausgebildet ist, daß die Duplikate (R1; R2; R3) verarbeitbar und ausgebbar sind,
wobei die Empfängersteuereinheit ferner so ausgebildet ist, daß sie je eine Prüf codierung eines Duplikats (R1; R2; R3) ermittelt, wobei sie aus einem Vergleich der Prüfcodierung mit dem Inhalt des Duplikats (R1; R2; R3) bestimmt, ob ein fehlerfreies Duplikat (R1; R2; R3) vorliegt, und wobei sie bei einem Empfang eines fehlerbehafteten Duplikats das betreffende Duplikat verwirft,
wobei der Empfänger ferner so ausgebildet ist, daß er eine Versetzungsregel für eine zeitliche Versetzung zwischen Datenpaketen (F1; F2; F3) und Duplikaten aus wenigstens einer den Datenpaketen (F1; F2; F3) und/oder deren Duplikaten (R1; R2; R3) entnommenen Versetzungsinformation bestimmt und unter Verwendung der Versetzungsregel die Duplikate (R1; R2; R3) selektiv auswertet.

6. Empfänger nach Anspruch 5,
**dadurch gekennzeichnet, daß**
die Versetzungsinformation aus einer vorgegebenen Zählfolge generiert wird, und zwar unter Verwendung eines Zählerstands der Zählung von empfangenen Datenpaketen (F1; F2; F3).

7. Empfänger nach einem der Ansprüche 5 bis 6,
**dadurch gekennzeichnet, daß**
die Empfangseinheit (15) von der Empfängersteuereinheit zwischen einem eingeschalteten Zustand und einem Zustand mit verringertem Energieverbrauch umschaltbar ist,
wobei der Empfänger die Empfangseinheit (15) zu Zeitpunkten, an denen ein Empfang eines Datenpakets (F1; F2; F3) und/oder eines Duplikats (R1; R2; R3) zu erwarten ist, in eingeschaltetem Zustand hält, und zu Zeitpunkten, an denen kein Empfang zu erwarten ist, in dem Zustand mit verringertem Energieverbrauch hält.

8. Empfänger nach Anspruch 7,
**dadurch gekennzeichnet, daß**
der Empfänger so ausgebildet ist, daß die Empfangseinheit (15) von der Empfängersteuereinheit selektiv für eine vorgegebene Zeit ausgeschaltet wird.

9. Empfänger nach einem der Ansprüche 5 bis 8,
**dadurch gekennzeichnet, daß**
der Empfänger so ausgebildet ist, daß er aus je einem Datenpaket (F1; F2; F3) bzw. aus je einem Duplikat (R1; R2; R3) eine dem Sender (2; 3; 4) des Datenpakets (F1; F2; F3) bzw. des Duplikats (R1; R2; R3) zugeordnete Identitätsinformation dekodiert und den zeitlichen Abstand zwischen zwei Datenpaketen (F1; F2; F3) mit übereinstimmenden Identitätsinformationen bestimmt.

10. Empfänger nach einem der Ansprüche 5 bis 9,
**dadurch gekennzeichnet, daß**
der Empfänger so ausgestaltet ist, daß er zum Bestimmen der Zeitpunkte für einen zu erwartenden Eingang eines Datenpakets (F1; F2; F3) ein von der Empfangseinheit (15) an die Empfängersteuereinheit abgegebenes Signal in einem Suchmodus wiederholt selektiv auswertet.

11. Empfänger nach Anspruch 10,
**dadurch gekennzeichnet, daß**
der Empfänger so ausgebildet ist, daß er Zeitpunkte für einen zu erwartenden Eingang je eines Datenpakets (F1; F2; F3) errechnet, und zwar aus einem Anfangszeitpunkt beim Eingang eines ersten Datenpakets sowie aus einer vorbestimmten, aus dem Inhalt oder der zeitlichen Lage des ersten Datenpakets und aus der aus dem ersten Datenpaket rekonstruierbaren Abstandszeit zwischen zwei aufeinanderfolgenden Datenpaketen.

12. Empfänger nach Anspruch 10,
**dadurch gekennzeichnet, daß**
der Empfänger so ausgebildet ist, daß er Zeitpunkte für einen zu erwartenden Eingang je eines Datenpakets aus einer Abstandszeit zwischen zwei Datenpaketen errechnet, und zwar aus einem Anfangszeitpunkt beim Eingang eines ersten Datenpakets sowie aus einem Wiederholungszeitpunkt beim Eingang eines weiteren Datenpakets.

13. Funkstrecke mit wenigstens einem Sender (2; 3; 4) gemäß einem der Ansprüche 1 bis 4 und mit wenigstens einem Empfänger (5) gemäß einem der Ansprüche 5 bis 12.

14. Kombiniertes Sende-/Empfangsmodul für eine Funkstrecke,
wobei das Sende-/Empfangsmodul einen Sender gemäß einem der Ansprüche 1 bis 4 und einen Empfänger gemäß einem der Ansprüche 5 bis 12 aufweist.

15. Kombiniertes Sende-/Empfangsmodul nach Anspruch 14,
**dadurch gekennzeichnet, daß**
der Sender und der Empfänger zur Vermeidung von Kollisionen von Datenpaketen und/oder deren Duplikaten betätigbar miteinander verbunden sind.

16. Kombiniertes Sende-/Empfangsmodul nach Anspruch 15,
**dadurch gekennzeichnet, daß**
der Empfänger des Sende-/Empfangsmoduls so ausgebildet ist, daß er beim Abtasten eines von einem weiteren Sender gesendeten Datenpakets den Sender des Sende/Empfangsmoduls zeitweilig unterdrückt oder zum Aussenden von Datenpaketen mit verändertem zeitlichen Abstand veranlaßt.

17. Verfahren zum Aussenden eines Datenpakete (F1; F2; F3) aufweisenden Datenstroms für eine Funkstrecke (1) mit wenigstens einem Sender (2; 3; 4) und mit wenigstens einem Empfänger (5) zur Übertragung eines aus Datenpaketen (F1; F2; F3) bestehenden Datenstroms, wobei das Verfahren das regelmäßig wiederholte Ausführen der folgenden Schritte aufweist:
- Bereitstellen eines zu übertragenden Datenpakets (F1; F2; F3),
- Einfügen einer sich aus dem Inhalt des jeweiligen Datenpakets (F1; F2; F3) ergebenden vorbestimmten Prüfcodierung in das jeweilige Datenpaket (F1; F2; F3),
- Aussenden des Datenpakets (F1; F2; F3),
- Erstellen eines Duplikats (R1; R2; R3) des Datenpakets (F1; F2; F3) sowie Einfügen jeweils einer Typinformation in das Datenpaket (F1; F2; F3) und/oder in das Duplikat (R1; R2; R3),
- Vorbestimmen einer Versetzungsinformation für eine zeitliche Versetzung zwischen Datenpaket (F1; F2; F3) und Duplikat (R1; R2; R3)sowie Einfügen der Versetzungsinformation in wenigstens ein Datenpaket (F1; F2; F3) und/oder in in wenigstens ein Duplikat (R1; R2; R3),
- Aussenden wenigstens eines Duplikats (R1; R2; R3), wobei das Aussenden des Duplikats (R1; R2; R3) bezüglich des Datenpakets (F1; F2; F3) gemäß der Versetzungsinformation zeitlich versetzt erfolgt und wobei sich die zeitliche Versetzung zwischen dem jeweiligen Datenpaket (F1; F2; F3) und dessen Duplikat (R1; R2; R3) gemäß einer vorbestimmten Versetzungsregel aus der Versetzungsinformation ergibt, wobei die Versetzungsinformation aus einer vorgegebenen Zählfolge generiert wird, und zwar unter Verwendung eines Zählerstands der Zählung der gesendeten Datenpakete (F1; F2; F3).

18. Verfahren zum Aussenden eines Datenstroms nach Anspruch 17,
**dadurch gekennzeichnet, daß**
zur Ausführung wenigstens ein Sender (2; 3; 4) verwendet wird, wobei je vom Sender (2; 3; 4) ausgesendetes Datenpaket (F1; F2; F3) bzw. dessen Duplikat (R1; R2; R3) mit einer dem Sender (2; 3; 4) zugeordneten Identitätsinformation versehen wird.

19. Verfahren zum Empfangen eines gemäß dem Anspruch 17 erzeugten, Datenpakete (F1; F2; F3) aufweisenden Datenstroms bei einer Funkstrecke (1) mit wenigstens einem Sender (2; 3; 4) und mit wenigstens einem Empfänger (5) , wobei das Verfahren einen Such-Modus sowie einen Übertragungsmodus aufweist,
wobei der Such-Modus die folgenden Schritte aufweist:
- Abtasten des Datenstroms auf das Vorhandensein von Datenpaketen (F1; F2; F3),
- Vorbestimmen von Zeitpunkten für einen zu erwartenden Eingang je eines Datenpakets (F1; F2; F3),
und wobei der Übertragungsmodus die folgenden Schritte aufweist:
- selektives Auswerten je eines Datenpakets (F1; F2; F3),
wobei aus einem Vergleich der Prüfcodierung mit dem Inhalt des Datenpakets (F1; F2; F3) bestimmt wird, ob ein fehlerfreies oder ein fehlerbehaftetes empfangenes Datenpaket (F1; F2; F3) vorliegt,
wobei der Such-Modus ferner die folgenden Schritte aufweist:
- Abtasten des Datenstroms auf das Vorhandensein von zu den Datenpaketen (F1; F2; F3) gehörigen Duplikaten (R1; R2; R3),
- Bestimmen einer Versetzungsregel für eine zeitliche Versetzung zwischen Datenpaketen (F1; F2; F3) und Duplikaten (R1; R2; R3) aus wenigstens einer den Datenpaketen (F1; F2; F3) und/oder deren Duplikaten (R1; R2; R3) entnommenen Versetzungsinformation,
und wobei der Übertragungsmodus für den Fall, daß ein fehlerbehaftetes Datenpaket vorliegt, den Schritt des selektiven Auswertens eines zu dem Datenpaket gehörenden Duplikats aufweist.

20. Verfahren zum Empfangen eines Datenstroms nach Anspruch 19, **dadurch gekennzeichnet, daß**
die Versetzungsinformation aus einer vorgegebenen Zählfolge generiert wird, und zwar unter Verwendung eines Zählerstands der Zählung der empfangenen Datenpakete (F1; F2; F3).

21. Verfahren zum Empfangen eines Datenstroms nach einem der Ansprüche 19 bis 20,
**dadurch gekennzeichnet, daß**
aus wenigstens einem Datenpaket (F1; F2; F3) eine dem Sender (2; 3; 4) des Datenpakets (F1; F2; F3) zugeordnete Identitätsinformation dekodiert wird, wobei der zeitliche Abstand zwischen zwei Datenpaketen mit übereinstimmenden Identitätsinformationen bestimmt wird.

22. Verfahren zum Empfangen eines Datenstroms nach einem der Ansprüche 19 bis 21,
**dadurch gekennzeichnet, daß**
aus wenigstens einem Datenpaket (F1; F2; F3) bzw aus wenigstens einem Duplikat eine dem Sender (2; 3; 4) des Datenpakets (F1; F2; F3) oder des Duplikats zugeordnete Zdentitätsinformation dekodiert wird, wobei eine Versetzungsregel für eine zeitliche Versetzung zwischen Datenpaketen (F1; F2; F3) und Duplikaten (R1; R2; R3) aus wenigstens einer von Datenpaketen (F1; F2; F3) und/oder deren Duplikaten entnommenen Versetzungsinformation bestimmt wird.

23. Verfahren zum Empfangen eines Datenstroms nach einem der Ansprüche 19 bis 22,
**dadurch gekennzeichnet, daß**
im Suchmodus der Schritt des Bestimmens des Zeitpunkts für einen zu erwartenden Eingang eines Datenpakets (F1; F2; F3) vorgesehen ist, bei dem ein von der Empfangseinheit (15) an die Empfängersteuereinheit abgegebenes Signal wiederholt selektiv ausgewertet wird.

24. Verfahren nach Anspruch 23,
**dadurch gekennzeichnet, daß**
Zeitpunkte für einen zu erwartenden Eingang je eines Datenpakets (F1; F2; F3) aus einem Anfangszeitpunkt, zu dem der Eingang eines ersten Datenpakets registriert wird, sowie aus einer vorbestimmten, aus dem Inhalt oder der zeitlichen Lage des ersten Datenpakets rekonstruierten oder aus dem ersten Datenpaket entnommenen Abstandszeit zwischen zwei Datenpaketen errechnet werden.

25. Verfahren nach Anspruch 23,
**dadurch gekennzeichnet, daß**
Zeitpunkte für einen zu erwartenden Eingang je eines Datenpakets aus einer Abstandszeit zwischen zwei Datenpaketen errechnet werden, wobei die Abstandszeit aus einem Anfangszeitpunkt, zu dem der Eingang eines ersten Datenpakets registriert wird, sowie aus einem Wiederholungszeitpunkt, zu dem der Eingang eines weiteren Datenpakets registriert wird, ermittelt wird.

26. Verfahren zum Betreiben einer Funkstrecke (1), das die Schritte eines Verfahrens zum Aussenden eines Datenpakete (F1; F2; F3) und Duplikate (R1; R2; R3) der Datenpakete (F1; F2; F3) aufweisenden Datenstroms gemäß einem der Ansprüche 17 bis 18 und die Schritte eines Verfahrens zum Empfangen eines Datenstroms gemäß einem der Ansprüche 19 bis 25 aufweist.

27. Fahrradcomputer mit wenigstens einem Sender gemäß einem der Ansprüche 1 bis 4 und mit einem Empfänger gemäß einem der Ansprüche 5 bis 12, wobei der Sender mit einem Sensor zur Messung von Raddrehungen, mit einem Sensor zur Messung von Kurbeldrehungen und/oder mit einem Sensor zur Messung des Fahrtwiderstandes verbunden ist und wobei der Empfänger ein Display zur Anzeige des vom Sensor übertragenen Werts aufweist.

28. Fitnesscomputer mit wenigstens einem Sender gemäß einem der Ansprüche 1 bis 4 und mit einem Empfänger gemäß einem der Ansprüche 5 bis 12, wobei der Sender mit einem Sensor zur Messung des Herzschlags und/oder der Schrittfrequenz eines Benutzers verbunden ist, und wobei der Empfänger ein Display zur Anzeige des vom Sensor übertragenen Werts aufweist.

## Claims

1. A transmitter (2; 3; 4) for a radio link (1) with at least one transmitter and with at least one receiver, in which a data stream consisting of data packets (F1; F2; F3) can be transmitted, the transmitter (2; 3; 4) comprising the following features:
- an input unit for inputting one data packet each,
- a transmitter control unit for processing the data packet and for inserting a predetermined test coding, resulting, in particular, from the content of the data packet, into the data packet, and
- a transmitting unit (6; 9; 12), which can be operated by the transmitter control unit, for transmitting the data packet, whereby the transmitter (2; 3; 4) is constructed in such a manner that it transmits the data packets (F1; F2; F3) regularly in succession,
whereby the transmitter control unit being constructed in such a manner that it generates at least one duplicate (R1; R2; R3) of the data packet, the data packet (F1; F2; F3) and/or the duplicate (R1; R2; R3) in each case comprising a type information and
whereby the transmitter (2; 3; 4) being constructed in such a manner that it transmits one data packet (F1; F2; F3) each and at least one duplicate (R1; R2; R3) regularly in succession, and the time offset between one data packet (F1; F2; F3) each and its duplicate (R1; R2; R3) varies in accordance with a predetermined offset rule and in accordance with an offset information provided in at least one data packet (F1; F2; F3) and/or in at least one duplicate,
whereby the transmitter is further constructed such that it generates the offset information from a predetermined counting sequence, particularly by using a count of the counting of data packets transmitted by the transmitter (2; 3; 4).

2. The transmitter as claimed in claim 1,
**characterized in that**
the transmitter is constructed such that it provides one data packet (F1; F2; F3) each or its duplicate (R1; R2; R3) with an identity information which is assigned to the transmitter (1, 2, 3).

3. The transmitter as claimed in claim 2,
**characterized in that**
the identity information comprises an information about the type and/or about the significance of the data packets and/or about at least a part of an unambiguous information about the transmitter.

4. The transmitter as claimed in one of the preceding claims,
**characterized in that**
the transmitting unit (6; 9; 12) can be switched between a switched-on state and a state with reduced energy consumption by the transmitter control unit, and wherein the transmitting unit (6; 9; 12) is constructed such that it is selectively held in the switched-on state at times at which data packets (F1; F2; F3) or duplicates (R1; R2; R3) are transmitted, and that it is held in the state with reduced energy consumption at times at which no data packets (F1; F2; F3) or duplicates (R1; R2; R3) are transmitted.

5. A receiver (5) for a radio link (1) with at least one transmitter (1, 2, 3), particularly as claimed in one of claims 1 to 4, in which a data stream consisting of data packets (F1; F2; F3) and duplicates (R1; R2; R3) of the data packets can be transmitted, the receiver (5) comprising the following features:
- a receiving unit (15) for receiving data packets (F1; F2; F3),
- a receiver control unit, connected to the receiving unit (15), by means of which the received data packets (F1; F2; F3) can be processed and output,
whereby the receiver control unit being constructed in such a manner that
- it determines a test coding each of a data packet (F1; F2; F3) and determines from a comparison of the test coding with the content of the data packet (F1; F2; F3) whether an error-free or an errored received data packet (F1; F2; F3) is present, wherein, when an errored data packet is received, it discards the relevant data packet,
- it determines times for an expected reception each of one data packet (F1; F2; F3),
- it selectively evaluates successive data packets by using a time interval between two data packets,
whereby the receiving unit (15) is constructed in such a manner that duplicates (R1; R2; R3) of the data packets (F1; F2; F3) can be received and that the receiver control unit is constructed in such a manner that the duplicates (R1; R2; R3) can be processed and output,
whereby the receiver control unit is further constructed in such a manner that it determines a test coding each of a duplicate (R1; R2; R3), whereby it determines from a comparison of the test coding with the content of the duplicate (R1; R2 ; R3) whether an error-free duplicate (R1; R2; R3) is present and wherein, when an errored duplicate is received, it discards the relevant duplicate,
whereby the receiver is further constructed such that it determines an offset rule for a time offset between data packets (F1; F2; F3) and duplicates from at least one offset information taken from the data packets (F1; F2; F3) and/or their duplicates (R1: R2; R3) and that it selectively evaluates the duplicates (R1; R2; R3) by using the offset rule.

6. The receiver as claimed in claim 5,
**characterized in that**
the offset information item is generated from a predetermined counting sequence, particularly by using a count of the counting of received data packets (F1; F2; F3).

7. The receiver as claimed in one of claims 5 to 6,
**characterized in that**
the receiving unit (15) can be switched between a switched-on state and a state with reduced energy consumption by the receiver control unit, wherein the receiver holds the receiving unit (15) in the switched-on state at times at which a reception of a data packet (F1; F2; F3) and/or of a duplicate (R1; R2; R3) can be expected and holds said receiving unit in the state with reduced energy consumption at times at which no reception can be expected.

8. The receiver as claimed in claim 7,
**characterized in that**
the receiver is constructed in such a manner that the receiving unit (15) can be selectively switched off for a predetermined time by the receiver control unit.

9. The receiver as claimed in one of claims 5 to 8,
**characterized in that**
the receiver is constructed in such a manner that it decodes from one data packet (F1; F2; F3) each or from one duplicate (R1; R2; R3) each, an identity information allocated to the transmitter (2; 3; 4) of the data packet (F1; F2; F3) or of the duplicate (R1; R2; R3), and determines the time interval between two data packets (F1; F2; F3) with matching identity informations.

10. The receiver as claimed in one of claims 5 to 9,
**characterized in that**,
for determining the times of an expected reception of a data packet (F1; F2; F3), it repetitively selectively evaluates a signal output by the receiving unit (15) to the receiver control unit in a search mode.

11. The receiver as claimed in claim 10,
**characterized in that**
the receiver is constructed in such a manner that it calculates times for an expected reception of one data packet (F1; F2; F3) each, in particular from a starting time on reception of a first data packet and from a predetermined interval time between two successive data packets, which can be reconstructed from the content or the position in time of the first data packet and from the first data packet.

12. The receiver as claimed in claim 10,
**characterized in that**
the receiver is constructed in such a manner that it calculates times for an expected reception of one data packet each from an interval time between two data packets, in particular from a starting time on reception of a first data packet and from a retransmission time on reception of a further data packet.

13. A radio link with at least one transmitter (2; 3; 4) as claimed in one of claims 1 to 4 and with at least one receiver (5) as claimed in one of claims 5 to 12.

14. A combined transceiver module for a radio link, the transceiver module comprising a transmitter as claimed in one of claims 1 to 4 and a receiver as claimed in one of claims 5 to 12.

15. The combined transceiver module as claimed in claim 14,
**characterized in that**
the transmitter and the receiver are operably connected to one another in order to prevent collisions of data packets and/or of their duplicates.

16. The combined transceiver module as claimed in claim 15,
**characterized in that**
the receiver of the transceiver module is constructed in such a manner that it temporarily suppresses the transmitter of the transceiver module during the sampling of a data packet transmitted by another transmitter or causes it to transmit data packets with changed time interval.

17. A method for transmitting a data stream comprising data packets (F1; F2, F3) for a radio link (1) with at least one transmitter (2; 3; 4) and with at least one receiver (5) for transmitting a data stream consisting of data packets (F1; F2; F3), the method comprising the regularly repeated execution of the following steps:
- providing a data packet (F1; F2; F3) to be transmitted,
- inserting a predetermined test coding, resulting, in particular, from the content of the respective data packet (F1 ; F2; F3), into the respective data packet (F1; F2; F3),
- transmitting the data packet (F1; F2; F3),
- generating a duplicate (R1; R2; R3) of the data packet (F1; F2; F3) and inserting in each case a type information into the data packet (F1; F2; F3) and/or into the duplicate (R1; R2; R3),
- predetermining an offset information item for a time offset between data packet (F1; F2; F3) and duplicate (R1; R2; R3) and inserting the offset information into at least one data packet (F1; F2; F3) and/or into at least one duplicate (R1; R2; R3),
- transmitting at least one duplicate (R1; R2; R3), the duplicate (R1; R2; R3) being transmitted offset in time in accordance with the offset information with respect to the data packet (F1; F2; F3) and the time offset between the respective data packet (F1; F2; F3) and its duplicate (R1; R2; R3) being obtained from the offset information in accordance with a predetermined offset rule, the offset information being generated from a predetermined counting sequence, particularly by using a count of the counting of the transmitted data packets (F1; F2; F3) .

18. The method for transmitting a data stream as claimed in claim 17,
**characterized in that**
for its execution, at least one transmitter (2; 3; 4) is used, each data packet (F1; F2; F3) transmitted by the transmitter (2; 3; 4) or its duplicate (R1; R2; R3) being provided with an identity information allocated to the transmitter (2; 3; 4).

19. The method for receiving a data stream generated in accordance with claim 17 and comprising data packets (F1; F2; F3) for a radio link (1) with at least one transmitter (2; 3; 4) and with at least one receiver (5), the method comprising a search mode and a transmission mode,
the search mode comprising the following steps:
- sampling the data stream for the presence of data packets (F1; F2; F3),
- predetermining times for an expected reception of one data packet (F1; F2; F3) each,
and the transmission mode comprising the following steps:
- selective evaluating of one data packet each (F1; F2; F3), it being determined from a comparison of the test coding with the content of the data packet (F1; F2; F3) whether an error-free or an errored received data packet (F1; F2; F3) is present,
the search mode further comprising the following steps
- sampling the data stream for the presence of duplicates (R1 ; R2; R3) belonging to the data packets (F1; F2; F3),
- predetermining an offset rule for a time offset between data packets (F1; F2; F3) and duplicates (R1; R2; R3) from at least one offset information taken from the data packets (F1; F2; F3) and/or their duplicates (R1; R2; R3),
and wherein the transmission mode comprising the step of selectively evaluating of a duplicate belonging to the data packet in the case where an errored data packet is present.

20. The method for receiving a data stream as claimed in claim 19, **characterized in that**
the offset information item is generated from a predetermined counting sequence, particularly by using a count of the counting of the received data packets (F1; F2; F3).

21. The method for receiving a data stream as claimed in one of claims 19 to 20,
**characterized in that**
from at least one data packet (F1; F2; F3), an identity information allocated to the transmitter (2; 3, 4) of the data packet (F1; F2; F3) is decoded, wherein the time interval between two data packets with matching identity information items is determined.

22. The method for receiving a data stream as claimed in one of claims 19 to 21,
**characterized in that**
from at least one data packet (F1; F2; F3) or from at least one duplicate, an identity information item allocated to the transmitter (2; 3; 4) of the data packet (F1; F2; F3) or of the duplicate is decoded, an offset rule for a time offset between data packets (F1; F2; F3) and duplicates (R1; R2; R3) being determined from at least one offset information item taken from data packets (F1; F2; F3) and/or from their duplicates.

23. The method for receiving a data stream as claimed in one of claims 19 to 22,
**characterized in that**
in the search mode, the step of determining the time of an expected reception of a data packet (F1; F2; F3) is provided at which a signal output by the receiving unit (15) to the receiver control unit is repeatedly selectively evaluated.

24. The method as claimed in claim 23,
**characterized in that**
times for an expected reception of one data packet (F1; F2; F3) each are calculated from a starting time at which the reception of a first data packet is registered, and from a predetermined interval time between two data packets, reconstructed from the content or the positioning in time of the first data packet or taken from the first data packet.

25. The method as claimed in claim 23,
**characterized in that**
times for an expected reception of one data packet each are calculated from an interval time between two data packets, the interval time being determined from a starting time at which the reception of a first data packet is registered and from a retransmission time at which the reception of a further data packet is registered.

26. A method for operating a radio link (1) which comprises the steps of a method for transmitting a data stream comprising data packets (F1; F2; F3) and duplicates (R1; R2; RS) of the data packets (F1; F2; F3) as claimed in one of claims 17 to 18 and the steps of a method for receiving a data stream as claimed in one of claims 19 to 25.

27. A bicycle computer with at least one transmitter as claimed in one of claims 1 to 4 and/or with a receiver as claimed in one of claims 5 to 12, wherein the transmitter is connected to a sensor for measuring wheel rotations, to a sensor for measuring pedal rotations and/or to a sensor for measuring the resistance to movement, and the receiver comprises a display for indicating the value transmitted by the sensor.

28. A fitness computer with at least one transmitter as claimed in one of claims 1 to 4 and/or with a receiver as claimed in one of claims 5 to 12, wherein the transmitter is connected to a sensor for measuring the heartbeat and/or the stepping rate of a user and the receiver comprises a display for indicating the value transmitted by the sensor.

## Revendications

1. Émetteur (2 ; 3 ; 4) pour une liaison radioélectrique (1) comprenant au moins un émetteur et comprenant au moins un récepteur et qui permet de transmettre un flux de données composé de paquets de données (F1 ; F2 ; F3), l'émetteur (2 ; 3 ; 4) présentant ici les caractéristiques suivantes :
- un module d'entrée pour l'entrée à chaque fois d'un paquet de données,
- un module de commande de l'émetteur pour traiter le paquet de données et pour insérer dans le paquet de données un code de contrôle prédéfini résultant du contenu du paquet de données, et
- un module d'émission (6 ; 9 ; 12) qui peut être commandé par le module de commande de l'émetteur pour émettre le paquet de données, l'émetteur (2 ; 3 ; 4) étant ici configuré de manière à émettre les paquets de données (F1 ; F2 ; F3) régulièrement l'un derrière l'autre, le module de commande de l'émetteur étant ici configuré de manière à créer au moins une copie (R1 ; R2 ; R3) du paquet de données, le paquet de données (F1 ; F2 ; F3) et/ou la copie (R1 ; R2 ; R3) présentant à chaque fois une information de type et l'émetteur (2 ; 3 ; 4) étant configuré de manière à émettre à chaque fois un paquet de données (F1 ; F2 ; F3) et au moins une copie (R1 ; R2 ; R3) régulièrement l'un derrière l'autre et le décalage dans le temps entre à chaque fois un paquet de données (F1 ; F2 ; F3) et sa copie (R1 ; R2 ; R3) variant en fonction d'une règle de décalage prédéfini et d'une information de décalage prévue dans au moins un paquet de données (F1 ; F2 ; F3) et/ou dans au moins une copie, l'émetteur étant en outre configuré de manière à générer l'information de décalage à partir d'une série de chiffres prédéfinie, et ce en utilisant l'état d'un compteur qui compte les paquets de données envoyés par l'émetteur (2 ; 3 ; 4).

2. Émetteur selon l'une des revendications précédentes, **caractérisé en ce que** l'émetteur est configuré de manière à joindre à chaque paquet de données (F1 ; F2 ; F3) ou à sa copie (R1 ; R2 ; R3) une information d'identité associée à l'émetteur (1, 2, 3).

3. Émetteur selon la revendication 2, **caractérisé en ce que** l'information d'identité présente une information sur la nature et/ou la signification des paquets de données et/ou au moins une partie d'une information explicite sur l'émetteur.

4. Émetteur selon l'une des revendications précédentes, **caractérisé en ce que** le module d'émission (6 ; 9 ; 12) du module de commande de l'émetteur peut être commuté entre un état actif et un état à consommation d'énergie réduite et le module d'émission (6 ; 9 ; 12) étant ici configuré de telle façon qu'il soit maintenu dans l'état actif de manière sélective aux moments où sont émis des paquets de données (F1 ; F2 ; F3) ou des copies (R1 ; R2 ; R3) et maintenu dans l'état à consommation d'énergie réduite aux moments où aucun paquet de données (F1 ; F2 ; F3) ou copies (R1 ; R2 ; R3) ne sont envoyés.

5. Récepteur (5) pour une liaison radioélectrique (1) comprenant au moins un émetteur (1, 2, 3), notamment selon l'une des revendications 1 à 4, avec lequel peut être transmis un flux de données composé de paquets de données (F1 ; F2 ; F3) et de copies (R1 ; R2 ; R3) des paquets de données, le récepteur (5) présentant ici les caractéristiques suivantes :
- un module de réception (15) pour la réception des paquets de données (F1 ; F2 ; F3),
- un module de commande du récepteur relié au module de réception (15) et avec lequel les paquets de données (F1 ; F2 ; F3) reçus peuvent être traités et restitués,
le module de commande du récepteur étant configuré de telle manière
- qu'il détermine à chaque fois un code de contrôle d'un paquet de données (F1 ; F2 ; F3) et détermine, à partir de la comparaison du code de contrôle avec le contenu du paquet de données (F1 ; F2 ; F3), si le paquet de données (F1 F2 ; F3) reçu est dépourvu d'erreur ou contient des erreurs, le paquet de données concerné étant refusé en cas de réception d'un paquet de données contenant des erreurs,
- qu'il détermine les moments d'une entrée attendue à chaque fois d'un paquet de données (F1 ; F2 ; F3),
- qu'il évalue de manière sélective les paquets de données successifs en utilisant un intervalle de temps entre deux paquets de données,
le module de réception (15) étant configuré de manière à pouvoir recevoir des copies (R1 ; R2 ; R3) des paquets de données (F1 ; F2 ; F3) et à ce que le module de commande du récepteur soit configuré de manière à ce que. les copies (R1 ; R2 ; R3) puissent être traitées et délivrées,
le module de commande du récepteur étant de plus configuré de manière à déterminer à chaque fois un code de contrôle d'une copie (R1 ; R2 ; R3) et déterminant, à partir de la comparaison du code de contrôle avec le contenu de la copie (R1 ; R2 ; R3), si la copie (R1 ; R2 ; R3) reçue contient des erreurs, la copie concernée étant refusée en cas de réception d'une copie contenant des erreurs,
le récepteur étant de plus configuré de manière à déterminer une règle de décalage pour un décalage dans le temps entre les paquets de données (F1 ; F2 ; F3) et les copies d'au moins l'un des paquets de données (F1 ; F2 ; F3) et/ou une information de décalage prélevée de leurs copies (R1 ; R2 ; R3) et évalue les copies (R1 ; R2 ; R3) de manière sélective en utilisant la règle de décalage.

6. Récepteur selon la revendication 5, **caractérisé en ce que** l'information de décalage est générée à partir d'une série de chiffres prédéfinie, et ce en utilisant l'état d'un compteur qui compte les paquets de données (F1 ; F2 ; F3) reçus.

7. Récepteur selon l'une des revendications 5 à 6, **caractérisé en ce que** le module de réception (15) du module de commande du récepteur peut être commuté entre un état actif et un état à consommation d'énergie réduite, le récepteur du module de réception (15) étant ici maintenu dans l'état actif aux moments où peut avoir lieu la réception d'un paquet de données (F1 ; F2 ; F3) ou d'une copie (R1 ; R2 ; R3) et maintenu dans l'état à consommation d'énergie réduite aux moments où aucune réception n'est attendue.

8. Récepteur selon la revendication 7, **caractérisé en ce que** le récepteur est configuré de telle manière que le module de réception (15) est désactivé de manière sélective par le module de commande du récepteur pendant une durée prédéfinie.

9. Récepteur selon l'une des revendications 5 à 8, **caractérisé en ce que** le récepteur est configuré de manière à décoder une information d'identité associée à l'émetteur (2 ; 3 ; 4) du paquet de données (F1 ; F2 ; F3) ou de la copie (R1 ; R2 ; R3) à chaque fois à partir d'un paquet de données (F1 ; F2 ; F3) ou à chaque fois à partir d'une copie (R1 ; R2 ; R3) et détermine l'écart dans le temps entre deux paquets de données (F1 ; F2 ; F3) dont les informations d'identité coïncident.

10. Récepteur selon l'une des revendications 5 à 9, **caractérisé en ce que** le récepteur est configuré de manière à évaluer, pendant la réception attendue d'un paquet de données (F1 F2 ; F3), de manière répétitive et sélective dans un mode de recherche un signal délivré par le module de réception (15) au module de commande du récepteur.

11. Récepteur selon la revendication 10,
**caractérisé en ce que** le récepteur est configuré de manière à calculer les moments d'une réception attendue à chaque fois d'un paquet de données (F1 ; F2 ; F3), et ce à partir d'un moment de début lors de l'entrée d'un premier paquet de données, à partir du contenu ou de la position dans le temps du premier paquet de données et à partir de l'intervalle de temps entre deux paquets de données successifs qu peut être reconstitué à partir du premier paquet de données.

12. Récepteur selon la revendication 10, **caractérisé en ce que** le récepteur est configuré de manière à calculer les moments d'une entrée attendue à chaque fois d'un paquet de données à partir d'un intervalle de temps entre deux paquets de données, et ce à partir d'un moment de début lors de l'entrée d'un premier paquet de données et à partir d'un moment de répétition lors de l'entrée d'un autre paquet de données.

13. Liaison radioélectrique comprenant au moins un émetteur (2 ; 3 ; 4) selon l'une des revendications 1 à 4 et comprenant au moins un récepteur (5) selon l'une des revendications 5 à 12.

14. Module combiné d'émission/réception pour une liaison radioélectrique, le module d'émission/réception présentant ici un émetteur selon l'une des revendications 1 à 4 et un récepteur selon l'une des revendications 5 à 12.

15. Module combiné d'émission/réception selon la revendication 14, **caractérisé en ce que** l'émetteur et le récepteur sont reliés entre eux de manière commandée afin d'éviter les collisions des paquets de données et/ou de leurs copies.

16. Module combiné d'émission/réception selon la revendication 15, **caractérisé en ce que** le récepteur du module d'émission/réception est configuré de manière à désactiver temporairement l'émetteur du module d'émission/réception en cas de détection d'un paquet de données envoyé par un autre émetteur ou lui commande d'émettre des paquets de données avec un intervalle de temps modifié.

17. Procédé d'émission d'un flux de données présentant des paquets de données (F1 ; F2 ; F3) pour une liaison radioélectrique (1) comprenant au moins un émetteur (2 ; 3 ; 4) et comprenant au moins un récepteur (5) pour transmettre un flux de données composé de paquets de données (F1 ; F2 ; F3), le procédé présentant l'exécution répétitive régulière des étapes suivantes :
- mise à disposition d'un paquet de données (F1 ; F2 ; F3) à transmettre,
- insertion dans le paquet de données (F1 ; F2 ; F3) d'un code de contrôle prédéfini résultant du contenu du paquet de données (F1 ; F2 ; F3) correspondant,
- émission du paquet de données (F1 ; F2 ; F3),
- création d'une copie (R1 ; R2 ; R3) du paquet de données (F1; F2 ; F3) et insertion à chaque fois d'une information de type dans le paquet de données (F1 ; F2 ; F3) et/ou dans la copie (R1 ; R2 ; R3),
- prédétermination d'une information de décalage pour un décalage dans le temps entre le paquet de données (F1 ; F2 ; F3) et la copie (R1 ; R2 ; R3) et insertion de l'information, de décalage dans au moins un paquet de données (F1 ; F2 ; F3) et/ou dans au moins une copie (R1 ;R2 ; R3),
- émission d'au moins une copie (R1 ; R2 ; R3), l'émission de la copie (R1 ; R2 ; R3) étant ici effectuée avec un retard dans le temps par rapport au paquet de données (F1 ; F2 ; F3) conformément à l'information de décalage et le décalage dans le temps entre les différents paquets de données (F1 ; F2 ; F3) et leurs copies (R1 ; R2 ; R3) étant obtenu à partir d'une règle de décalage prédéfinie résultant de l'information de décalage, l'information de décalage étant ici générée à partir d'une suite de chiffres prédéfinie, et ce en utilisant l'état d'un compteur qui compte les paquets de données (F1 ; F2 ; F3) envoyés.

18. Procédé d'émission d'un flux de données selon la revendication 17, **caractérisé en ce qu'**au moins un émetteur (2, 3, 4) est utilisé pour la réalisation, les paquets de données (F1 ; F2 ; F3) à chaque fois envoyés par l'émetteur (2 ; 3 ; 4) ou leurs copies (R1 ; R2 ; R3) étant accompagnés d'une information d'identité affectée par l'émetteur (2 ; 3 ; 4).

19. Procédé de réception d'un flux de données généré selon la revendication 17 et présentant des paquets de données (F1 ; F2 ; F3) avec une liaison radioélectrique (1) comprenant au moins un émetteur (2 ; 3 ; 4) et comprenant au moins un récepteur (5), le procédé présentant un mode de recherche ainsi qu'un mode de transmission,
le mode de recherche présentant ici les étapes suivantes :
- examen du flux de données pour y détecter la présence de paquets de données (F1 ; F2 ; F3),
- prédéfinition des moments pour une entrée attendue à chaque fois d'un paquet de données (F1 ; F2 ; F3),
et le mode de transmission présentant ici les étapes suivantes :
- évaluation sélective à chaque fois d'un paquet de données (F1 ; F2 ; F3), une comparaison du code de contrôle avec le contenu du paquet de données (F1 ; F2 ; F3) servant ici à déterminer si le paquet de données (F1 ; F2 ; F3) reçu est dépourvu d'erreur ou contient des erreurs,
le mode de recherche comprenant ici en plus les étapes suivantes :
- examen du flux de données pour y détecter la présence de copies (R1 ; R2 ; R3) associées aux paquets de données (F1 ; F2 ; F3),
- détermination d'une règle de décalage pour un décalage dans le temps entre les paquets de données (F1 ; F2 ; F3) et les copies (R1 ; R2 ; R3) à partir d'une information de décalage prélevée au moins dans l'un des paquets de données (F1 ; F2 ; F3) et/ou dans leurs copies (R1 ; R2 ; R3),
et le mode de transmission, en présence d'un paquet de données contenant des erreurs, présentant l'étape d'évaluation sélective d'une copie associée au paquet de données.

20. Procédé de réception d'un flux de données selon la revendication 19, **caractérisé en ce que** l'information de décalage est générée à partir d'une suite de chiffres prédéfinie, et ce en utilisant l'état d'un compteur qui compte les paquets de données (F1 ; F2 ; F3) reçus.

21. Procédé de réception d'un flux de données selon l'une des revendications 19 à 20, **caractérisé en ce qu'**une information d'identité associée à l'émetteur (2 ; 3 ; 4) du paquet de données (F1 ; F2 ; F3) est décodée à partir d'au moins un paquet de données (F1 ; F2 ; F3), l'écart dans le temps entre deux paquets de données étant déterminé avec des informations d'identité coïncidentes.

22. Procédé de réception d'un flux de données selon l'une des revendications 19 à 21, **caractérisé en ce qu'**une information d'identité associée à l'émetteur (2 ; 3 ; 4) du paquet de données (F1 ; F2 ; F3) ou de la copie est décodée à partir d'au moins un paquet de données (F1 ; F2 ; F3) ou à partir d'au moins une copie, une règle de décalage pour un décalage dans le temps entre les paquets de données (F1 ; F2 ; F3) et les copies (R1 ; R2 ; R3) étant déterminée à partir d'une information de décalage prélevée d'au moins l'un des paquets de données (F1 ; F2 ; F3) et/ou de leurs copies.

23. Procédé de réception d'un flux de données selon l'une des revendications 19 à 22, **caractérisé en ce qu'**en mode de recherche il est prévu l'étape de détermination du moment d'une entrée attendue d'un paquet de données (F1 ; F2 ; F3), étape au cours de laquelle est évalué de manière répétitive et sélective un signal délivré par le module de réception (15) au module de commande du récepteur.

24. Procédé selon la revendication 23, **caractérisé en ce que** les moments d'une réception attendue à chaque fois d'un paquet de données (F1 ; F2 ; F3) sont calculés à partir d'un moment de début auquel est enregistrée l'entrée d'un premier paquet de données, ainsi qu'à partir d'un intervalle de temps prédéfini entre deux paquets de données, reconstitué à partir du contenu ou de la position dans le temps du premier paquet de données ou prélevé dans le premier paquet de données.

25. Procédé selon la revendication 23, **caractérisé en ce que** les moments d'une réception attendue à chaque fois d'un paquet de données sont calculés à partir d'un intervalle de temps entre deux paquets de données, l'intervalle de temps étant ici déterminé à partir d'un moment de début où est enregistrée l'entrée d'un premier paquet de données ainsi qu'à partir d'un moment de répétition où est enregistrée l'entrée d'un autre paquet de données.

26. Procédé pour faire fonctionner une liaison radioélectrique (1) qui présente les étapes d'un procédé d'émission d'un flux de données présentant des paquets de données (F1 ; F2 ; F3) et des copies (R1 ; R2 ; R3) des paquets de données (F1 ; F2 ; F3) selon l'une des revendications 17 à 18 et les étapes d'un procédé de réception d'un flux de données selon l'une des revendications 19 à 25.

27. Ordinateur pour vélo comprenant au moins un émetteur selon l'une des revendications 1 à 4 et comprenant un récepteur selon l'une des revendications 5 à 12, l'émetteur étant relié à un capteur destiné à mesurer les rotations de la roue, à un capteur destiné à mesurer les rotations du pédalier et/ou à un capteur destiné à mesurer la résistance au déplacement et le récepteur présentant un écran pour afficher les valeurs transmises par le capteur.

28. Ordinateur de culture physique comprenant au moins un émetteur selon l'une des revendications 1 à 4 et comprenant un récepteur selon l'une des revendications 5 à 12, l'émetteur étant relié à un capteur destiné à mesurer les battements du coeur et/ou la fréquence des pas d'un utilisateur et le récepteur présentant un écran pour afficher les valeurs transmises par le capteur.
